# EUROPEAN PATENT APPLICATION

(11) **EP 0 960 942 A2**
(43) Date of publication of application: **01.12.1999**
(21) Application number: 99201593.3
(22) Date of filing: 20.05.1999
(51) Int. Cl.: C12N 15/87, C12N 15/86, C12N 7/04

(54) **Targeted delivery through a cationic amino acid transporter**

(30) Priority: 20.05.1998 EP 98201693
(71) Applicant: Introgene B.V., 2333 AL Leiden (NL)
(72) Inventor: Van Es, Helmuth, 2133 DJ Hoofddorp (NL); Verlinden, Stefan, 2312 WB Leiden (NL); Havenga, Menzo, 2401 BV Alphen aan den Rijn (NL)
(74) Representative: Ottevangers, Sietse Ulbe

(57) **Abstract**

The invention relates to the targeted delivery of substances to cells. The invention provides a virus-like particle or gene delivery vehicle provided with a ligand capable of binding to a human amino acid transporter. Provided are for example ligands that can bind to the human transporter of cationic L-amino acids (hCAT1). Such hCAT1 binding molecules find applications in the design of vector systems for entry into human or primate cells. Preferred are retroviral envelope molecules, which - when incorporated in a virus particle - can infect hCAT1 positive cells at high frequencies. Also within the scope of the invention are methods for the design of such hCAT1 binding molecules.

## Description

The invention relates to the targeted delivery of substances to cells.

Delivery of substances to cells allows specific treatment of said cells with compounds that act in the targeted cell. For example, tumour cells, when targeted with toxic components, selectively die when said toxin is delivered to said cell. Yet other cells, when provided with a gene lacking in said cell, can be restored in their function, so-called gene therapy.

Delivery of a compound to a cells preferably occurs with a vehicle or particle that effectively brings the compound to the desired cell or cells and than delivers said compound into that cell (in vivo or in vitro) where it can exert its action. For this purpose, for example particles such as virus-like particles are suited. These particles, often derived from known viruses, such as retrovirus or adenovirus, are small enough to penetrate in-between tissues and cells and arrive at a cell of choice where it for example can fuse with said cell and deliver its compound. Said virus-like particles may or may not be infectious in themselves, their main concern is the targeted delivery of the compound of interest, such as a gene, a toxin or immunostimulating components such as antigens.

Yet other examples are gene-delivery vehicles, specifically designed to transfer a gene to a cell of interest. Virus-like particles capable of delivering a gene are examples of said gene-delivery vehicles, however, also other examples of such vehicles, of non-viral origin, such as liposomes or microbodies, or even latex particles, are known. Vehicles such as liposomes or microbodies can of course also carry other compounds than a gene, in particular toxic or immunostimulating components such as antigens can be included in such a vehicle.

These vehicles or particles all have in common that they need to be provided with a molecule or fragment thereof (ligand) capable of binding with said targeted cell, allowing targeting of said particle or vehicles to cells. There is a need for specific or broadly applicable ligands that react with cell-surface receptors on cells. In particular there is a need for ligands that react with cell-surface receptors after which efficient transfer of said compound to said cell, such as a gene, is possible. Especially in human medicine, such a ligand would enable better application of genetransfer therapy than is possible now.

It has been a long-standing objective to exploit retrovirus technology in human gene therapy applications. However, the infection spectrum of retroviruses limits the applications of these viruses in such applications. All known env variants have a rather broad infection spectrum in common. Here lies one of the major shortcomings of current recombinant retrovirus technology. For the purpose of gene therapy, retroviruses are very useful vehicles for the transfer of therapeutic sequences, if proper ligand-receptor targets were available. In conclusion, the concept of the use of retroviruses in human gene therapy is well documented (Gordon and Anderson, 1994; Havenga et al., 1997; Vile et al., 1996). However, it would be clearly advantageous and desirable to devise a strategy for targeted delivery of retroviruses, and modification of the infection spectrum.

The invention provides a virus-like particle or gene delivery vehicle provided with a ligand capable of binding to a human amino acid transporter. The invention provides said particle or vehicles wherein said ligand comprises peptide molecules or fragments thereof binding said transporter, for example to hCAT1. The peptides or fragments thereof can bind to for example the third extracellular domain of the cationic amino acid transporter hCAT1 or can bind to cells expressing this domain of hCAT1 protein on their extracellular cell surface. These hCAT1 binding molecules can be peptides or antibody fragments displayed on a filamentous phage or as free molecules. In a preferred embodiment, the invention provides a virus-like particle or gene delivery vehicle for delivery of genes to human cells, however, it is also possible to provide said particles or vehicles with other compounds, such as toxins for selective killing or antigens for immunisation.

In a particular embodiment of the invention, a virus-like particle or gene delivery vehicle is provided comprising at least one viral protein provided with said ligand. Included in the present invention is the use of hCAT1 binding ligands to provide a particle or vehicle that employs hCAT1 to enter a hemopoietic stem cell or any other cell expressing hCAT1. hCAT1 ligands can be incorporated in the envelope of a retrovirus or the capsid of any other viral or non-viral gene transfer vehicle such as an adenoviral vector. Incorporation of these hCAT1 binding sequences can be done using techniques known in the art.

The invention provides a virus-like particle or gene delivery vehicle wherein said viral protein comprises an envelope protein. In a preferred embodiment the invention provides a a mutant retroviral envelope that is derived from a wild-type ecotropic envelope and which employs hCAT1 to enter the human or primate cell by binding to hCAT1. Such a new retroviral envelope molecule, when incorporated in a retroviral virion, will be able to infect hCAT1 positive cells such as human PHSCs at high efficiencies. The mutant retroviral envelopes can be used to pseudotype recombinant type C retrovirus including but not limited to murine leukemia retroviral vectors. In a further embodiment of the present invention these hCAT1 binding envelopes can also be used to pseudotype lentiviral vectors including equine or HIV derived lentiviral vectors (Kim et al., 1998; Rizvi and Panganiban, 1992), (Kafri et al., 1997; Poeschla et al., 1996), (Miyoshi et al., 1997; Naldini et al., 1996b). Any hCAT1 ligannds or binding envelope molecules or parts thereof made according to the methods described herein or other methods can be ligated into full length mammalian retroviral envelope expression constructs and introduced in cell lines expressing and containing all the sequences necessary for the generation of infectious and functional retroviral particles, in a preferred embodiment the invention provides a virus-like particle or gene delivery vehicle derived from a retrovirus.

In yet another embodiment, the invention provides a virus-like particle or gene delivery vehicle wherein said viral protein comprises a capsid protein. hCAT1 binding sequences or ligand can also be incorporated in the capsid proteins of adenovirus including but not limited to the HI loop of the knob domain of an adenovirus (Krasnykh et al., 1998) preferably an adenovirus which does not bind to the adenoviral receptor CAR1 or MHC1. This results in an adenovirus that enters cells through hCAT1. Deduced from mCAT1 absent expression in mouse liver (Closs et al., 1993) an hCAT1 binding adenovirus does not exhibit liver transduction when administered *in vivo.* By combining an hCAT1 targeted knob with a ligand for another *in vivo* target hCAT1, targeting of an adenovirus can remove an important limitation of in vivo use of adenoviral vectors for gene therapy (Sullivan et al., 1997). In another embodiment an hCAT1 targeted adenovirus will more efficiently transduce cells that are difficult to transduce such as endothelial cells or smooth muscle cells as compared to a wildtype adenoviral vector including but not limited to an adenoviral vector derived from the adenoviral serotype 5, the invention provides a virus-like particle or gene delivery vehicle derived from an adenovirus.

An hCAT1 targeted adenovirus is useful for local applications of adenoviral vector such as in patients with restenosis following balloon angioplasty where smooth muscle cells need to be transduced with for example an adenoviral vector carrying the ceNOS cDNA. More efficient transduction of these tissues results in lower multiplicity's of infections (MOIs) that can be used and therefore less vector associated toxicity to the tissues surrounding the target cells (PCT/EP98/00723).

In a preferred embodiment, the invention provides a virus-like particle or gene delivery vehicle according to the invention wherein said amino acid transporter is a cationic amino acid transporter, preferably a human cationic amino acid transporter-1 (hCAT1). In a preferred example of the invention provided in the experimental part, the invention provides a virus-like particle or gene delivery vehicle wherein said ligand comprises an amino acid sequence selected from Table 2, preferably from the last four different sequences of Table 2 or a sequence functionally related thereto. Various examples of a ligand having hCAT1 binding activity are provided, a particularly strong example is a ligand comprising at least a part of, comprising minimally 5, more preferably minimally 7 amino acids of the amino acid sequence SVSVGMKPSPRP.

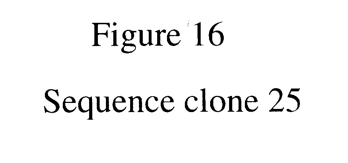

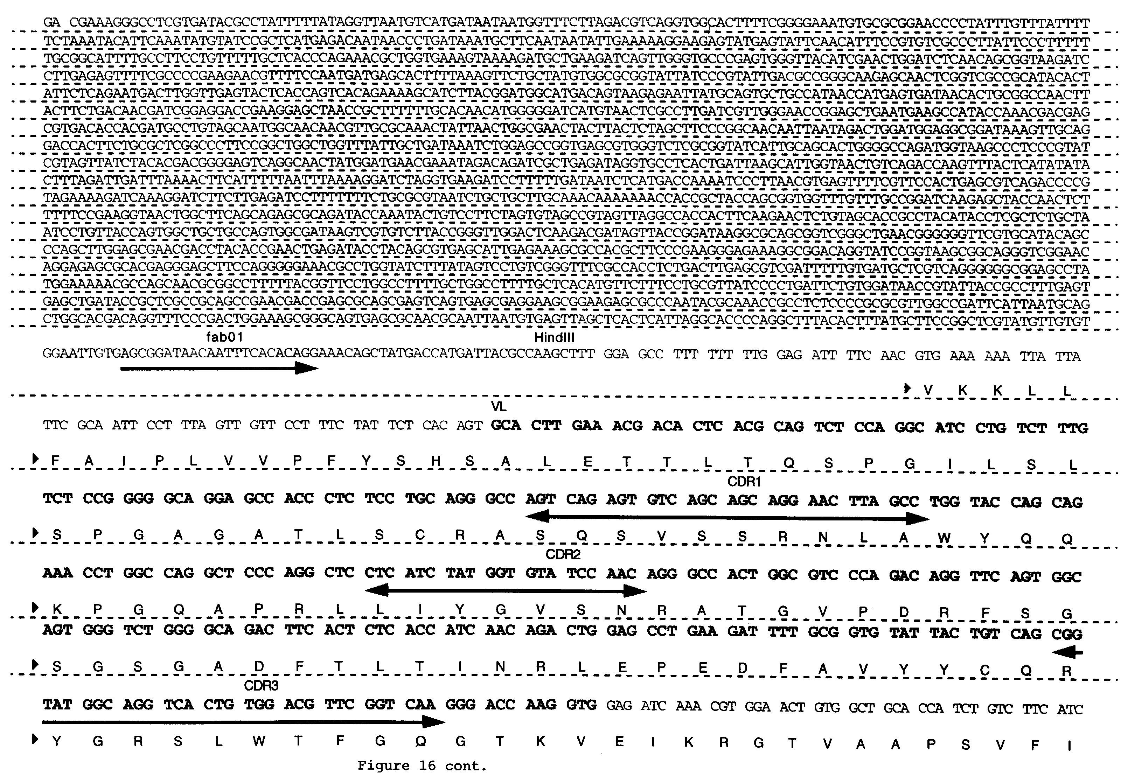

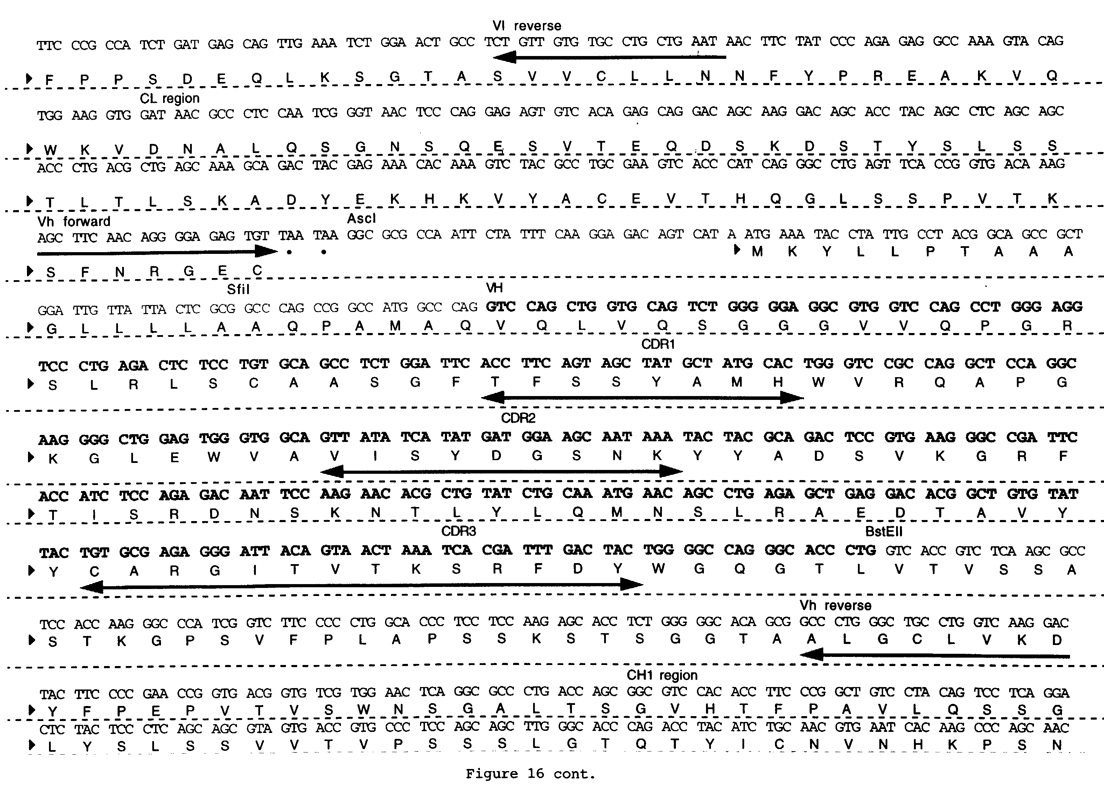

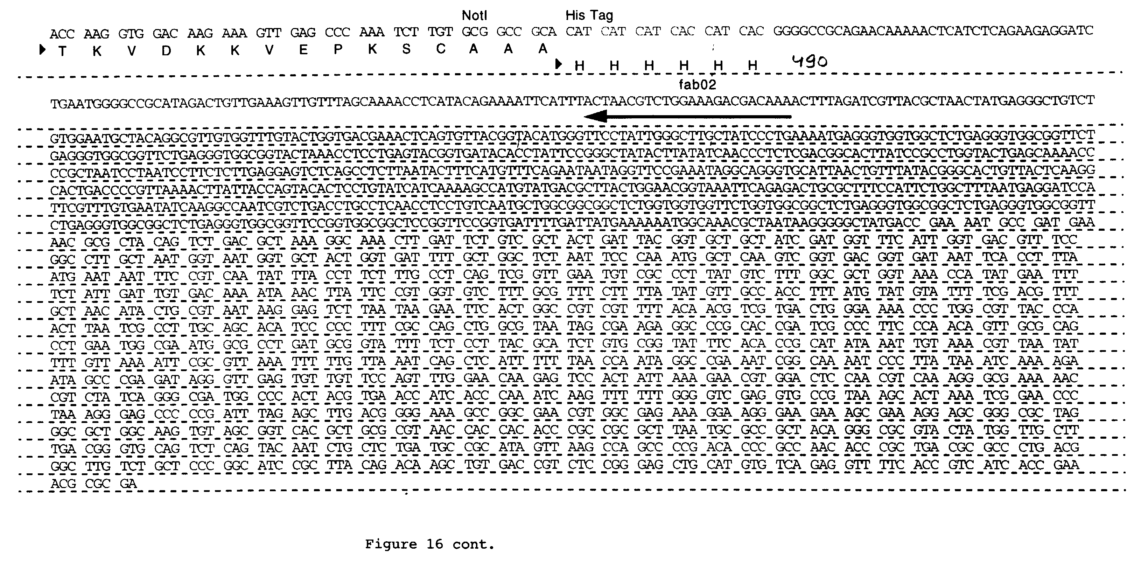

In yet another embodiment, the invention provides a virus-like particle or gene delivery vehicle according to the invention wherein wherein said ligand comprises a fragment derived from a phage displaying at least one antibody fragment selected for its capacity to bind with said amino acid transporter, in particular a virus-like particle or gene delivery vehicle is provided wherein said antibody fragment comprises an amino acid sequence as shown in Figure 16 or a an amino acid sequence functionally equivalent thereto or obtainable by a method as described in the experimental part of this description.

The invention also provides use of a virus-like particle or gene delivery vehicle according to the invention in genetransfer therapy. In numerous gene therapy applications targeted delivery of genes into defined cells is provided by the invention, most notably in the case of in vitro gene transfer into cell types present with low abundance in cell mixtures and in approaches for in vivo gene transfer into cells in a living animal body. In a particular embodiment, the particles or vehicles provided by the invention are used for gene therapy using hCAT1 mediated gene transfer including but not limited to mammalian smooth muscle cells or hemopoietic stem cells such as CD34+CD38- or CD34+(CD33CD38CD71)- cells, including but not limited to adenoviral or retroviral gene transfer vehicles.

The invention also provides a method for selecting a filamentous phage expressing a protein capable of binding to a ligand comprising constructing a phage library, enriching said library for phages having desired binding characteristics by at least one round of selection of phages for their capacity to bind to a synthetic peptide derived from said ligand, further comprising enriching said library for phages having desired binding characteristics by at least one round of selection of phages for their capacity to bind to a cell expressing said ligand.

The invention for example provides a peptide phage display to select hCAT1 binding peptides for incorporation in a ligand. To isolate peptides that bind to the third extracellular domain of we employed peptide phage display. A 12 mer peptide phage display library was purchased from New England Biolabs. This library is constructed in the filamentous E. coli phage m13 and the peptide sequences are displayed as N-terminal fusion proteins with the minor coat protein pIII. The unamplified library had a complexity of 1.9 x 10⁹ different sequences as determined by the suppliers. We amplified the library once before using it to select hCAT1 binding peptide phages. Two targets were used to select for peptide displaying phages which bind to the third extracellular domain of hCAT1. First the predicted third extracellular domain of hCAT1 was synthesised as a synthetic peptide by Neosystem, Strassbourg, France. The N-terminus of this peptide was biotinylated and followed by three amino acid linker residues KRR, followed by the predicted sequence of the third extracellular domain. Secondly we generated cell lines derived from the human 911 cell line that overexpress hCAT1 as judged by steady state mRNA expression levels. The hCAT1 expression construct hATRCC1 which is a pcDNA3 based expression construct of the hCAT1 cDNA was employed to transfect 911 cell lines followed by selection for neomycine resistance. A cloned cell line designated k08 was isolated which expresses high levels of hATRCC1 derived hCAT1 mRNA.

The invention is further described in the experimental part of this description which is not limiting the invention thereto.

### Experimental part

Retroviruses are RNA viruses which efficiently integrate their genetic information into the genomic DNA of infected cells via a reverse-transcribed DNA intermediate. This property of their life-cycle and the fact that parts of their genetic material can be replaced by foreign DNA sequences make retroviruses one of the most promising vectors for the delivery of genes in human gene therapy procedures, most notably for gene therapies which rely on gene transfer into dividing tissues. Most retroviral vector systems are based on mouse retroviruses and consist of two components, i.e. (i) the recombinant retroviral vector carrying the foreign sequences of interest, and (ii) so-called packaging cells expressing the structural viral proteins of which the encoding sequences are lacking in the retroviral vector. Expression of (i) in (ii) results in the production of recombinant retroviral particles capable of transducing susceptible target cells.

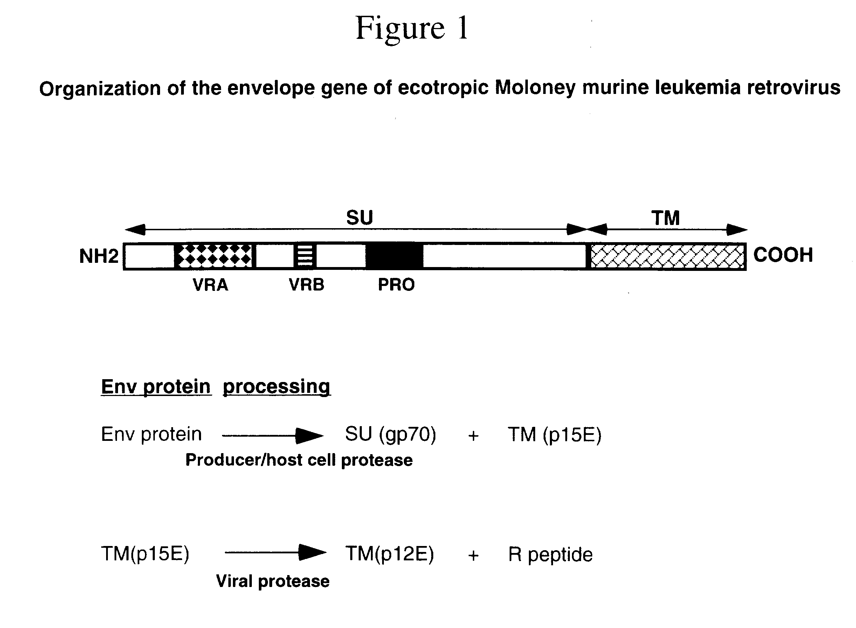

The infectivity and host cell range of the retrovirus particle is conferred by an envelope glycoprotein which specifically binds to a receptor molecule on the target cell membrane. The envelope glycoprotein of all known retroviruses consists of two associated peptides, which are derived by proteolytic cleavage from the same precursor protein encoded by the retroviral envelope (env) gene (Gunzburg and Salmons, 1996; Weiss, 1996). The amino terminal domain encompasses specific binding site(s) for its receptor on the target cell membrane, determining the virus host range. Within this domain of about 200 amino acids highly conserved sequences are present that are interrupted by two segments designated VRA and VRB which vary in sequence and length among various mammalian type C retroviruses (Battini et al., 1992). The carboxy terminal peptide, which contains trans-membrane anchor sequences, is assumed to account for the selective uptake of the envelope glycoprotein in the virus particle and to mediate fusion between the virus membrane and - depending on the type of virus - the plasma membrane or intracellular vesicle membrane of the target cell (Januszeski et al., 1997; Thomas et al., 1997). In figure 1 a schematic representation of the structure of MuLV env protein is given. Several envelope glycoprotein variants with different infection spectra for mammalian cells have been identified (Battini et al., 1992).

There are examples or recombinant viruses carrying an amphotropic or GaLV envelope. Recombinant viruses carrying an amphotropic or GaLV envelope are capable of infecting human and murine cells and are commonly used in gene transfer trials including human gene therapy involving the pluripotent hemopoietic stem cell (PHSC) (Havenga et al., 1997). Gene transfer frequencies into PHSCs of human patients and non human primate animal models have been shown to be extremely low and limit therapeutic stem cell gene therapy (Havenga et al., 1997; Hoogerbrugge et al., 1996; Van Beusechem et al., 1993; van Beusechem et al., 1992).

One important limiting factor has been shown to be low expression levels of retroviral receptors such as the one mediating entry of amphotropic MuLV retrovirus (GLVR2) (Orlic et al., 1996; van Es et al., 1996). The quiescent state of PHSCs when isolated for ex vivo gene transfer procedures poses another blockade (Knaan-Shanzer et al., 1996). Murine stem cell gene therapy experiments have traditionally been performed with ecotropic MuLV vectors (Havenga et al., 1997). Recombinant viruses carrying an ecotropic envelope are only capable of infecting murine cells. Transfer of genes into murine PHSCs using ecotropic retroviral vectors has been shown to result in high transduction efficiencies in circulating PHSC derived peripheral blood cells (PBL). The transduction efficiencies are high enough to be therapeutic if achieved in human PHSCs reaching levels of PHSC gene transfer varying between 30-80 %.

A small number of studies have been performed in which the transduction efficiency into murine PHSCs of ecotropic and amphotropic retroviruses were actually compared directly (Havenga et al., 1997). One of these studies indicated that infection with amphotropic retrovirus resulted in expression and thus transgene presence for less than 8 weeks whereas infection with ecotropic virus resulted in expression for more than 44 weeks after transplantation (Demarquoy, 1993). In a similar study, ecotropic virus was shown to be approximately 50 fold more efficient in transducing murine PHSCs as compared to amphotropic retrovirus (Orlic et al., 1996).

Ecotropic and amphotropic retrovirus differ in the receptor that is employed for virus entry (Albritton et al., 1989; van Zeijl et al., 1994). Ecotropic virus binds target cells via the ecotropic receptor mCAT1 which is a transporter of cationic L-amino acids (Kim et al., 1991) and amphotropic retrovirus binds target cells via the amphotropic receptor GLVR2, a sodium dependent phosphate transporter GLVR2 (Kavanaugh et al., 1994; Miller and Miller, 1994; van Zeijl et al., 1994).

A comparative study measuring mRNA levels of both the ecotropic and amphotropic receptors in mouse PHSCs (lin⁻ c-kitbright) revealed an important difference. This study demonstrated that ecotropic receptor (mCAT1) mRNA levels in these cells are high whereas amphotropic receptor (GLVR2) mRNA levels were undetectable by RT-PCR (Orlic et al., 1996). GLVR2 expression studies on CD34⁺(CD38,CD33,CD71)⁻(CD34⁺lin⁻ cells) isolated from human bone marrow, umbilical cord blood and immobilised peripheral blood supports these data (van Es et al., 1996).

Another important factor which plays a role in determining successful retroviral entry and integration is the postbinding route of entry of a retrovirus particle. The postbinding entry route for ecotropic virus is different from that of amphotropic retrovirus. Ecotropic retrovirus tranductions are sensitive to lysosomotropic agents such as chloroquine and NH4Cl. This suggests that upon binding of the ecotropic retrovirus, the retrovirus is internalised by receptor mediated endocytosis (McClure et al., 1990). In contrast upon binding of the envelope of amphotropic retrovirus the viral envelope directly fuses with the plasma membrane. This is a process that is not disrupted by lysosomotropic agents suggesting that the postbinding steps of amphotropic MuLV virus are essentially different from those of ecotropic MuLV retrovirus (McClure et al., 1990).

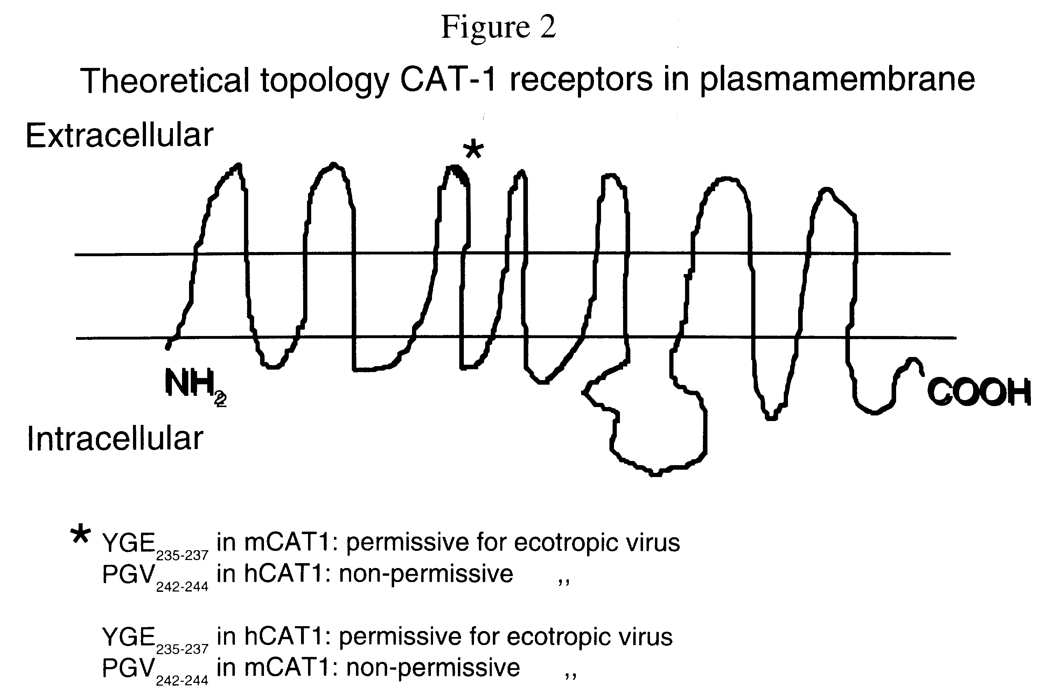

The human homologue of the murine ecotropic virus receptor mCAT1 is hCAT1. Like mCAT1 mRNA expression in mouse PHSCs, hCAT1 mRNA is expressed at high levels in human PHSCs (Orlic et al., 1996). For both mCAT1 and hCAT1 the normal function is the import of cationic amino acids such as lysine and arginine (Albritton et al., 1993; Malhotra et al., 1996). The third predicted extracellular domain of mCAT1 includes a sequence YGE. The residues are crucial for receptor function. In the nonfunctional hCAT1 the sequence of the third extracellular domain is PGV. Mutation of the human sequence into one or two of the residues of mCAT1 results in a hCAT1 protein with ecotropic receptor function (Albritton et al., 1993; Yoshimoto et al., 1993). See also figure 2.

A number of mutant ecotropic envelope molecules have been described in the literature. MacKrell et al have mutated amino acids within the receptor-binding domain VRA of ecotropic MuLV envelope in order to identify residues involved in receptor binding. Virions incorporating mutant envelopes carrying mutations at amino acid residue D84 have lost their binding capabilities to the ecotropic receptor mCAT1 (MacKrell et al., 1996). Virions carrying D84 mutated envelope protein were tested on human cells to search for a possible change in receptor recognition specificity but were found not to infect human cells (Mike Januszeski, personal communication).
Skov and Andersen have studied ecotropic Moloney envelope interactions with mCAT1 by generation of mutant envelope molecules with mutated arginine and lysine residues in gp70 including VRA followed by introduction in a replication competent retroviral backbone (Skov and Andersen, 1993).Mutations R135G, K137Q, R157G and R159A (R102G,K104Q,R124G and R126A without signal peptide respectively) resulted in virions that were not able to replicate.
Kingsman et al have described in PCT application WO96/31602 an insertion site in the VRA domain of ecotropic envelope which allows modification of the tropism. An integrin binding sequence was inserted resulting in infection of human cells expressing the respective integrin.

PVC-211 murine leukemia virus (MuLV) is a neuropathogenic variant of ecotropic Friend MuLV (F-MuLV) that causes a rapidly progressive neurodegenerative disease in susceptible rodents. PVC-211 MuLV, but not the parental F-MuLV, can infect rat brain capillary endothelial cells (BCEC) efficiently, and the major determinant for BCEC tropism of PVC-211 MuLV is localized within the env gene. More specific analysis indicated that E116G and E129K substitutions in the background of the F-MuLV envelope protein were sufficient for conferring BCEC tropism on the virus (Masuda et al., 1996a). Host range changes of these mutations were found to include CHO cells normally not infectable with ecotropic F-MuLV or M-MuLV. The latter suggests that these mutations overcome a negative effect of CAT1 CHO cell receptor glycosylation in the region of virus binding in the third extracellular domain of mCAT1 (Masuda et al., 1996b).

By employing particular natural env variants the transduction spectrum can be limited to some extend, but true specificity for human target cells of interest can not be obtained following this strategy (Masuda et al., 1996a; von Kalle et al., 1994; Wilson et al., 1994).

In the present invention we describe the expansion of the host range of an ecotropic retrovirus or other gene transfer vehicle such as an adenoviral vector resulting in increased transduction of hemopoietic stem cells. In this invention, targeted delivery is accomplished by directing the retrovirus particle to cell membrane molecules differing from the natural receptor. This could then lead to increased specificity of transduction.

The present invention discloses examples of molecules that bind to hCAT1 and that can be used to develop gene transfer vehicles such as retroviral and adenoviral vectors. In particular, the invention relates to proteins and derivatives thereof expressed in the lipid bilayer of enveloped virus particles such as retroviruses. Methods, materials, procedures and pharmaceutical formulations for the design and preparation of the above molecules and virus particles are also part of the invention. These molecules and virus particles have applications in the field of virology, gene therapy, biochemistry and molecular biology.

The present invention relates to peptide molecules binding to hCAT1. These molecules are characterized by their ability to bind the third extracellular domain of the cationic amino acid transporter hCAT1 either a synthetic peptide encompassing this third extracellular domain or by binding to cells expressing this domain of hCAT1 protein on their extracellular cell surface. These hCAT1 binding molecules can be peptides or antibody fragments displayed on a filamentous phage or as free molecules.

Included in the present invention are filamentous phages displaying hCAT1 binding molecules and that can be used to transfer genes into cells by modification of the phage genome using techniques known in the art.

Included in the present invention is the use of hCAT1 binding molecules to design vectors that employ hCAT1 to enter a HSC or any other cell expressing hCAT1. hCAT1 binding molecules can be incorporated in the envelope of a retrovirus or the capsid of any other viral or non-viral gene transfer vehicle such as an adenoviral vector. Incorporation of these hCAT1 binding sequences can be done using techniques known in the art.

Preferred are mutant retroviral envelopes that are derived from wild-type ecotropic envelope and which employ hCAT1 to enter the human or primate cell by binding to hCAT1. These new retroviral envelope molecules, when incorporated in a retroviral virion, will be able to infect hCAT1 positive cells such as human PHSCs at high efficiencies. The mutant retroviral envelopes can be used to pseudotype recombinant type C retrovirus including but not limited to murine leukemia retroviral vectors. In a further embodiment of the present invention these hCAT1 binding envelopes can also be used to pseudotype lentiviral vectors including equine or HIV derived lentiviral vectors (Kim et al., 1998; Rizvi and Panganiban, 1992), (Kafri et al., 1997; Poeschla et al., 1996), (Miyoshi et al., 1997; Naldini et al., 1996b).

Any hCAT1 binding envelope molecules or parts thereof made according to the methods described herein or other methods can be ligated into full length mammalian retroviral envelope expression constructs and introduced in cell lines expressing and containing all the sequences necessary for the generation of infectious and functional retroviral particles including but not limited to cell lines preferably derived from the adenoviral E1 transformed, human cell line PER.C6 (WO97/00326) and that express murine leukemia gag-pol constructs and a retroviral vector containing long terminal repeats (LTRs), and retroviral RNA packaging signals such as those vectors described in WO96/35798. The hCAT1 binding envelopes made according to the subject material of this invention can also be used to pseudotype vectors other than murine leukemia retroviral vectors including but not limited to lentiviral vectors (Naldini et al., 1996a; Naldini et al., 1996b).

In a further embodiment of the present invention, hCAT1 binding sequences can also be incorporated in the capsid proteins of adenovirus including but not limited to the HI loop of the knob domain of an adenovirus (Krasnykh et al., 1998) preferably an adenovirus which does not bind to the adenoviral receptor CAR1 or MHC1. This results in an adenovirus that enters cells through hCAT1. Deduced from mCAT1 absent expression in mouse liver (Closs et al., 1993) an hCAT1 binding adenovirus does not exhibit liver transduction when administered *in vivo*. By combining an hCAT1 targeted knob with a ligand for another *in vivo* target hCAT1 targeting of an adenovirus can remove an important limitation of in vivo use of adenoviral vectors for gene therapy (Sullivan et al., 1997). In another embodiment an hCAT1 targeted adenovirus will more efficiently transduce cells that are difficult to transduce such as endothelial cells or smooth muscle cells as compared to a wildtype adenoviral vector including but not limited to an adenoviral vector derived from the adenoviral serotype 5.

An hCAT1 targeted adenovirus is useful for local applications of adenoviral vector such as in patients with restenosis following balloon angioplasty where smooth muscle cells need to be transduced with for example an adenoviral vector carrying the ceNOS cDNA. More efficient transduction of these tissues results in lower multiplicity's of infections (MOIs) that can be used and therefore less vector associated toxicity to the tissues surrounding the target cells (PCT/EP98/00723).

In another aspect of the present invention, the hCAT1 binding human FAbs that are part of the subject matter of this invention can be used to measure expression of hCAT1 molecules on cells that are targets for gene therapy using hCAT1 mediated gene transfer including but not limited to mammalian hemopoetic stem cells such as CD34+CD38- or CD34+(CD33CD38CD71)- cells. This could be part of a procedure aimed at determining when or whether a patients cells are most susceptible to gene transfer through hCAT1 including but not limited to adenoviral or retroviral gene transfer vehicles.

The skilled artisan will be able to apply the teaching of the present invention to other virus capsid or envelope or non-viral gene transfer molecules or vehicles than those exemplified herein without departing from the present invention and therefore the examples presented are illustrations and not limitations. It is intended that all such other examples be included within the scope of the appended claims.

### Example 1. Sequences of hCAT1 cDNAs amplified from human CD34+ cells.

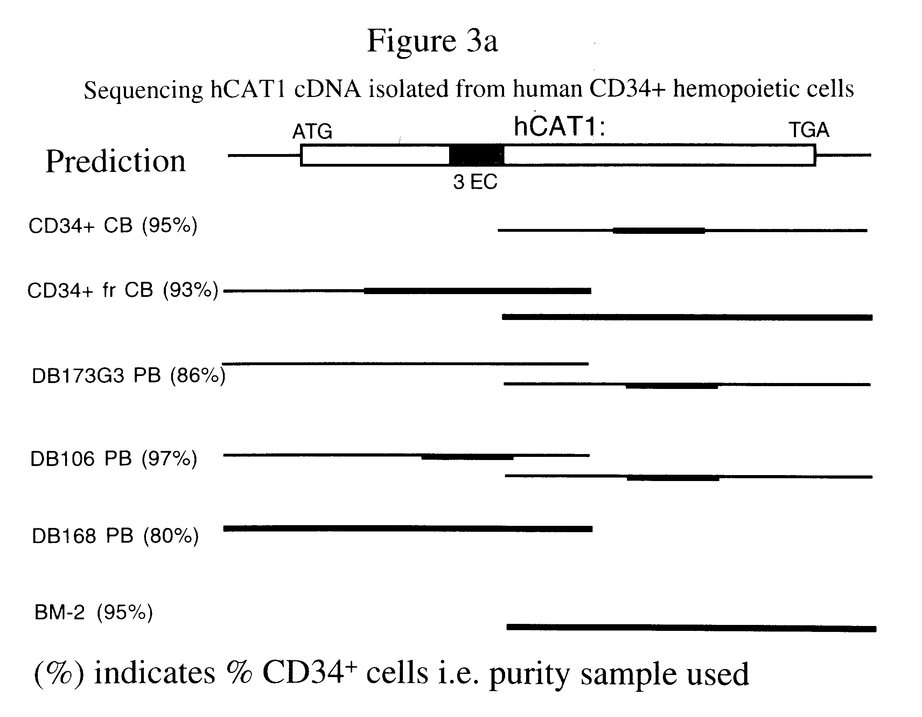

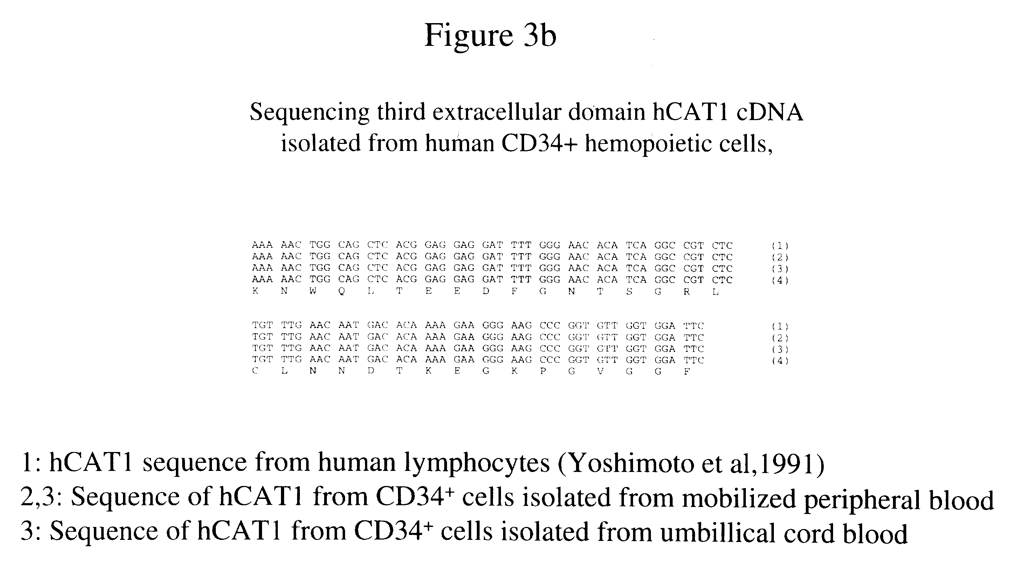

For the purpose of developing gene transfer tools that enter PHSCs through hCAT1, specifically through binding to the third extracellular domain, we isolated total RNA from a number of different human CD34⁺ samples and determined the cDNA sequence of hCAT1 (see figure 2). Total RNA was isolated according to the protocol described by Chomczynski et al (Chomczynski and Sacchi, 1987). RT-PCR was performed by using the SuperScript Preamplification System for First Strand cDNA Synthesis (Life Technologies). For first strand synthesis random hexamers were used. The hCAT1 cDNA was amplified with two sets of primers, each resulting in a product of approximately 1 kb encompassing the open reading frame of the hCAT1 mRNA (Yoshimoto et al., 1991). DNA sequencing was performed by BaseClear, Leiden, The Netherlands using automated sequence analysis. In figure 3a and 3b the results of sequence analysis of hCAT1 cDNA isolated from CD34⁺ cells from mobilized peripheral blood or umbilical cord blood are compiled. Clearly from these nucleotide sequence analyses (figure 3b ) it can be deduced that indeed in the CD34⁺ samples tested hCAT1 is expressed and includes the third extracellular domain with predicted sequence KNWQLTEEDFGNTSGRLCLNNDTKEGKPGVGGF which includes the sequence PGV determining function as receptor (see above). Therefore targeting through this domain or part of this domain of hCAT1 in hemopoietic CD34⁺ cells including but not limited to hemopoietic stem cells such as defined by lineage negative phenotypes e.g. CD34⁺CD38⁻ is possible.

### Example 2. Peptide phage display to select hCAT1 binding peptides.

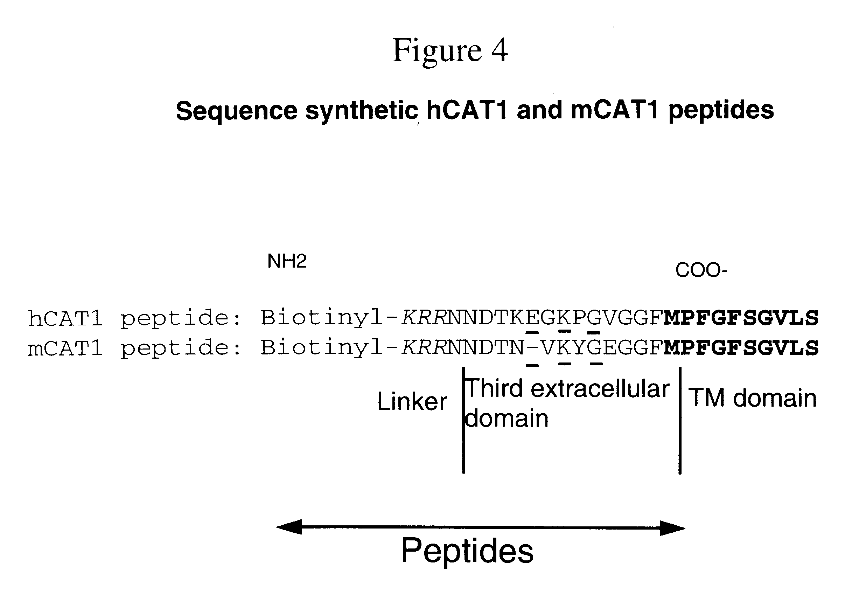

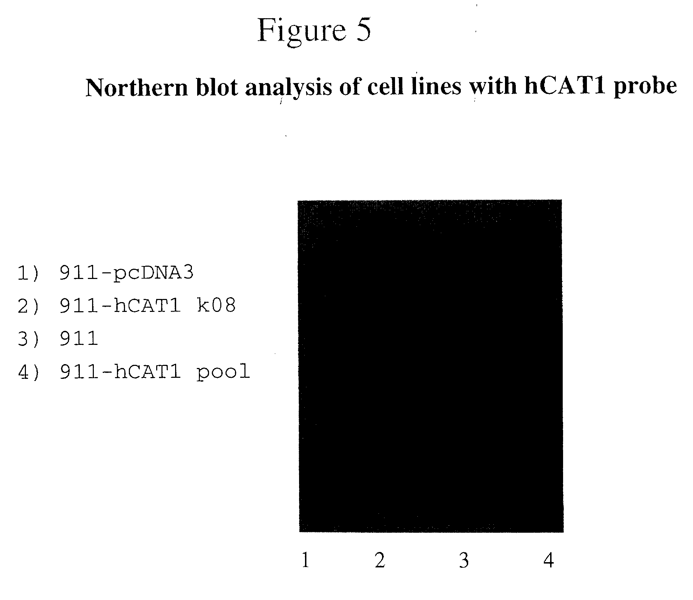

To isolate peptides that bind to the third extracellular domain of hCAT1 (Albritton et al., 1993) (figure 2) we employed peptide phage display. A 12 mer peptide phage display library was purchased from New England Biolabs. This library is constructed in the filamentous E. coli phage m13 and the peptide sequences are displayed as N-terminal fusion proteins with the minor coat protein pIII. The unamplified library had a complexity of 1.9 x 10⁹ different sequences as determined by the suppliers. We amplified the library once before using it to select hCAT1 binding peptide phages. Two targets were used to select for peptide displaying phages which bind to the third extracellular domain of hCAT1. First the predicted third extracellular domain of hCAT1 was synthesised as a synthetic peptide by Neosystem, Strassbourg, France. The N-terminus of this peptide was biotinylated and followed by three amino acid linker residues KRR, followed by the predicted sequence of the third extracellular domain (figure 4). Secondly we generated cell lines derived from the human 911 cell line that overexpress hCAT1 as judged by steady state mRNA expression levels. The hCAT1 expression construct hATRCC1 which is a pcDNA3 based expression construct of the hCAT1 cDNA (Malhotra et al., 1996)was employed to transfect 911 cell lines followed by selection for neomycine resistance in 1 mg/ml of G418 (Genetecin, Life Technologies, Inc). A cloned cell line designated k08 was isolated which expresses high levels of hATRCC1 derived hCAT1 mRNA (figure 5).

To select for peptide displaying phages that bind to the putative third extracellular domain of hCAT1 as expressed on human cells the following selection procedure was employed. Six rounds of selection on biotinylated hCAT1 peptide (figure 4) followed by three rounds of selection on hCAT1 overexpressing cells k08. Initially two separate selections were carried out differing in the stringency of washing. Low stringency washing consisted of 3 washes with 2 % (w/v) milk powder in PBS with 0.05 % (v/v) Tween 20 and 3 washes with PBS. High stringency washing consisted of 5 washes with 2 % (w/v) milk powder, PBS with 0.05 % Tween 20, 5 washes with PBS, 0.05 % Tween 20 and 5 washes with PBS. After 1 round of selection on 911-hCAT1-k08 cells eluted phages from both washing procedures were pooled and used for a second and third round of selection on 911-hCAT1-k08 cells. The results of these experiments are depicted in table 1. Clearly the ratio of input over output increases upon selection on hCAT1 peptide indicative of selection for binding phages. When selection on hCAT1 positive cells was started the ratio drops and slightly increases in the last round on hCAT1 expressing human cells.

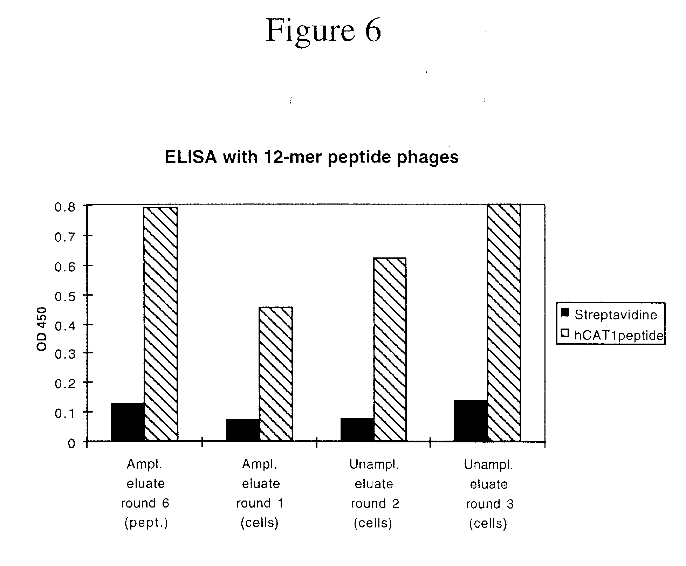

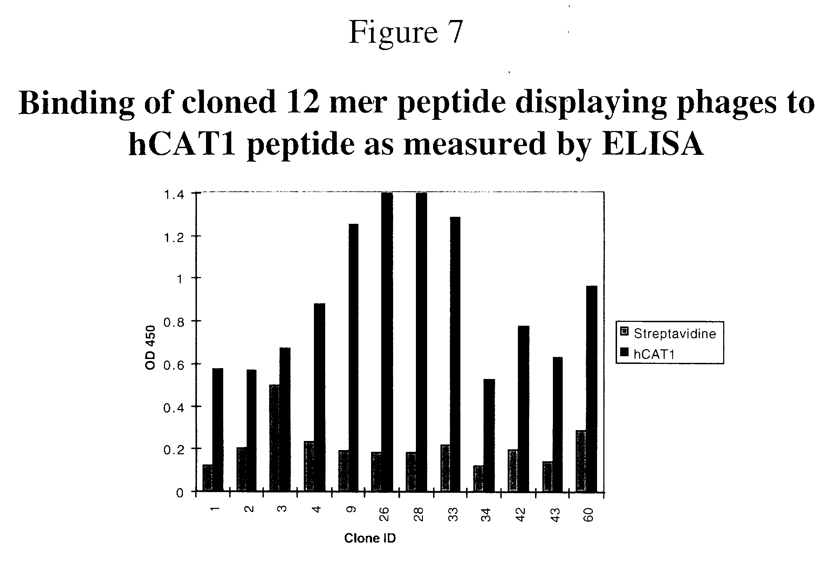

After the last round of selection with the hCAT1 peptide and after each round of cell selection the pools of peptide displaying phages were tested for binding to immobilized hCAT1 peptide using an Enzyme Linked Immunosorbent Assay (ELISA). 96-well plates were coated with 2 mg/ml biotinylated BSA in PBS and incubated for 1 hour 37 °C after which the wells were rinsed 3x for 5 minutes with PBS/0.05% Tween 20. Then the wells were saturated with streptavidin (10 mg/ml in PBS/0.5% gelatin) for 1 hour at room temperature (RT) and washed 3 times with PBS/0.05 % Tween 20. Then the wells were incubated overnight at 4 °C with biotinylated hCAT1 peptide (figure 4) at a concentration of 10 mg/ml in PBS. The next day the wells were rinsed two times with PBS/0.1 % Tween 20 and 2x with PBS. Then the wells were blocked with 2% non-fat milkpowder in PBS for at least 30 minutes at RT followed by three rinses with with PBS/0. 1 % Tween and three with PBS. Subsequently an equal volume of 4% non-fat milkpowder in PBS was added to all wells and culture supernatant or purified phage (PEG precipitated) and incubated for 1.5 hours at RT. After this incubation the wells were washed three times with PBS/0.1 % Tween 20 and three times with PBS followed by incubation with an anti-m13 antibody solution (Pharmacia, 1:5000 in 2% non-fat milkpowder in PBS) for 1 hour at RT. Again the wells were washed three times with PBS/0.1 % Tween and three times with PBS followed by the addition of a rabbit-anti goat HRP conjugate solution (BioRad, 1:2000 in 2% non-fat milkpowder in PBS) for 1 hour at RT. After this incubation the wells were washed again three times with PBS/0.1% Tween and three times with PBS. Detection of phage binding was then visualized using TMB colour solution (0.1 mg/ml TMB, 1% DMSO, 1x TMB buffer, 0.001% 30% H₂O₂ in H₂O) 20-30 min in the dark at RT and stopped with 2 N H₂SO₄ and read at 450 nm in a microplate reader. Using this hCAT1 specific ELISA an enrichment of phages binding to hCAT1 peptide is achieved (figure 6). Importantly after binding of the peptide selected pools to hCAT1 overexpressing cells eluted phages still bind to hCAT1 peptide. Clones isolated from round 3 on hCAT1 overexpressing cells were isolated and tested on hCAT1 peptide ELISA (figure 7). Except 1, all tested clones bound to hCAT1 peptide and thus to the third extracellular domain displayed on human cells.

To confirm enrichment for specific sequences and to determine the amino acid sequence of the 12 mer peptides displayed, we isolated single stranded m13 phage DNA for automated sequence analysis (Baseclear, Leiden, The Netherlands). The oligonucleotide used for sequencing was 5'-CCCTCATAGTTAGCGTAACG-3'. We sequenced clones isolated from the pools of various peptide and cell selections. For this purpose we pooled the eluates of the two different washing conditions. In addition to the amplified 12 mer peptide library we only selected clones from peptide rounds 3 ,5 and 6 and cell rounds 1,2 and 3. In table 2 the sequences determined for the various clones are given. Clearly a very strong selection occurred because all cell selected phage clones displayed one sequence namely: SVSVGMKPSPRP. This sequence is also displayed by phages in hCAT1 peptide selected pool 6 in a mixture with 3 other sequences. These other phages are lost once the phage pools selected on hCAT1 peptide are selected for binding to hCAT1 overexpressing cells.

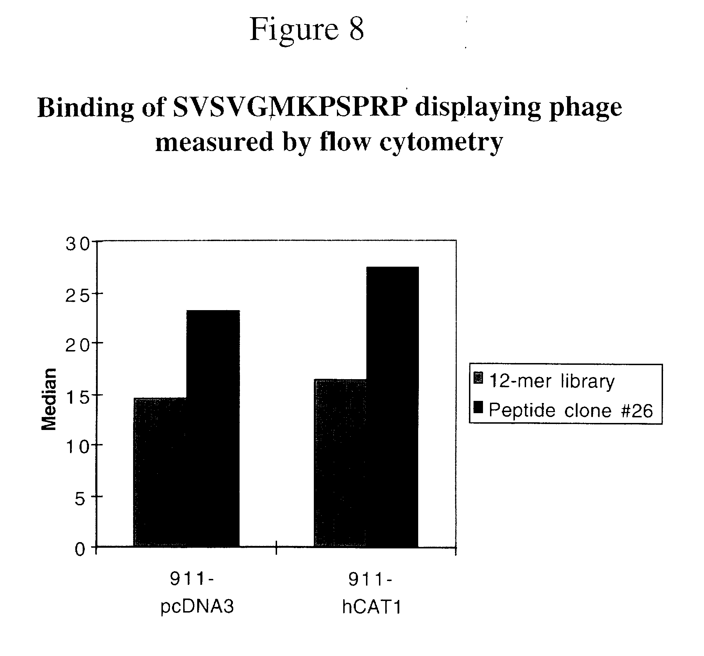

The cloned SVSVGMKPSPRP displaying m13 phage was used in experiments to measure binding of the displayed sequence to cells that express hCAT1. First we did an experiment using the flow cytometer and the 2 cell lines 911-pcDNA3 and 911-hCAT1(k08).Cells were incubated with 10¹¹ phage in 100 ml PBS/0.1% BSA for 1 hour at room temperature. Subsequently the cells were washed twice with PBS/0.1 % BSA followed by incubation of the cells with anti-m13 antibody (Pharmacia, 1:500 in PBS/0.1 % BSA) for 30 min at room temperature and washed twice with PBS/0.1 % BSA. Then the cells were incubated with rabbit-anti goat FITC (DAKO, 1:50 in PBS/0.1 % BSA) for 30 min at RT and washed twice with PBS/0.1 % BSA. Binding of phage was then measured in the FL1 channel of a Becton and Dickinson flow cytometer. As a control we used an identical amount of phage from the amplified 12 mer libary. In figure 8 the results of this experiment are depicted. Clone #26 phage binds to 911 cells and in particular to 911 cells that overexpress hCAT1.

We also measured cell binding of phage incubating hCAT1 expressing cells with phage followed by titering total cell bound phage, eluted phage and cell associated phage fractions on E.coli using a standard m13 plating assay on a lawn of E.coli cells. For this purpose E.coli strain ER2537 is grown overnight in LB medium. This overnight culture is then used to inoculate 20 ml of fresh LB medium at an OD₆₀₀ₙₘ of 0.05. Once at an OD₆₀₀ₙₘ of 0.5 500 ml of the ER2537 E.coli bacteria were mixed with 500 ml dilutions of phage samples and incubated at RT for 10 min. Plating on a standard LB-agar plate was performed by mixing 3 ml top agar with 200 ml of each sample. Once the topagar was solidified the plates were transferred upside down to a 37 °C incubator for 12-14 hours. Plaques were counted and used to determine the number of phage particles binding to hCAT1 expressing cells. In table 3 cell binding is depicted.

Clearly from these results we can conclude that the 12 mer peptide displaying phage with sequence SVSVGMKPSPRP indeed binds to hCAT1 expressing cells. hCAT1 expressing Cells were also incubated at 37 °C followed by elution of bound phages plus cell associated phages were liberated. Both were used in phage titering on E.coli and clearly a cell associated fraction is detected. This suggests that the phage displaying sequence SVSVGMKPSPRP and which bind to hCAT1 also enter a human hCAT1 expressing cell. This feature of sequence SVSVGMKPSPRP could be used for the development of gene transfer products useful in gene therapy.

### Example 3. Human FAb phage display to select hCAT1 binding human antibody molecules.

To isolate antibodies that bind to the third extracellular domain of hCAT1 (Albritton et al., 1993) (figure 2,4) we employed phages displaying human FAb fragments encompassing the light and heavy variable and constant regions. A human FAb phage display library was constructed in phage display vector pCES1 a vector derived from pCANTAB6 (McGuiness et al., 1996). The library was constructed in the filamentous E. coli phage m13 and the FAb sequences are displayed partly as N-terminal fusion proteins with the minor coat protein pIII. The unamplified library had a complexity of approximately 3.3 x 10¹⁰ different sequences. Two targets were used to select for peptide displaying phages which bind to the third extracellular domain of hCAT1. First the predicted third extracellular domain of hCAT1 was synthesized as a synthetic peptide by Neosystem, Strassbourg, France. The N-terminus of this peptide was biotinylated and followed by three amino acid linker residues KRR, followed by the predicted sequence of the third extracellular domain (figure 2,4). Secondly we generated cell lines derived from the human 911 cell line that overexpress hCAT1 as judged by steady state mRNA expression levels. The hCAT1 expression construct hATRCC1 which is a pcDNA3 based expression construct of the hCAT1 cDNA was employed to transfect 911 cell lines followed by selection for neomycine resistance in 1 mg/ml of G418 (Geneticin, Life Technologies, Inc). A cloned cell line designated k08 was isolated which expresses high levels of hATRCC1 derived hCAT1 mRNA (figure 5).

To select for FAb displaying phages that bind to the putative third extracellular domain of hCAT1 as expressed on human cells the following selection procedure was employed. Four rounds of selection on biotinylated hCAT1 peptide (figure 4) followed by three rounds of selection on hCAT1 overexpressing cells k08. For selection on biotinylated hCAT1 peptide 250 ml of FAb library (or eluted phage from the previous round) was mixed with 250 ml 4% Marvel in PBS and equilibrated while rotating at RT for 1 hour. Subsequently biotinylated hCAT1 peptide (20-500 nM in H₂O) was added. This mix was incubated on the rotator at RT for 1 hour before 250 ml equilibrated streptavidin-dynabeads in 2% Marvel in PBS was added. After incubation on a rotator at RT for 15 min the beads with the bound phage were washed 5 times with PBS/2% Marvel/0.1% Tween, 5 times with PBS /0.1% Tween and 5 times with PBS. Then the phage were eluted by incubation with 0.1M Tri-ethyl-amine on a rotator at RT for 10 min and neutralised in 1 M Tris-HCl pH 7.4. The eluted phage were titered and amplified in TG1 before the next selection. For selection on 911-hCAT1 cells, the cells were harvested at a confluency of about 80 % and suspended in PBS/10 % FBS/2 % Marvel to a final concentration of at least 3x10⁶ cells/ml. This cell suspension was incubated for 30 min on a rowing boat mixer (or rotator), while at the same time phage were also preincubated in PBS/10 % FBS/2 % Marvel. Then the cells were centrifuged, resuspended in the preincubated phage solution and incubated on a rowing boat mixer (or rotator) for 1 hour. Afterwards the cells were washed 10 times with PBS/10 % FBS/2 % Marvel and twice with PBS. The cells were centrifuged and resuspended in 0.6 ml water. Subsequently 0.6 ml 200 mM triethylamine was added (dropwise while vortexing). After 5 minutes the suspension was neutralised by adding 0.6 ml of 1 M Tris-HCl pH 7.4 (dropwise while vortexing).After cenrifugation (5 min, 14000 rpm) the supernatant was transferred to a new tube and titered and amplified in TG1 before the next selection. The results of these experiments are depicted in table 4. Clearly the ratio of input over output increases upon selection on hCAT1 peptide indicative of selection for hCAT1 peptide binding phages. When selection on hCAT1 positive cells was started the ratio dropped and slightly increased in the last round on hCAT1 expressing cells.

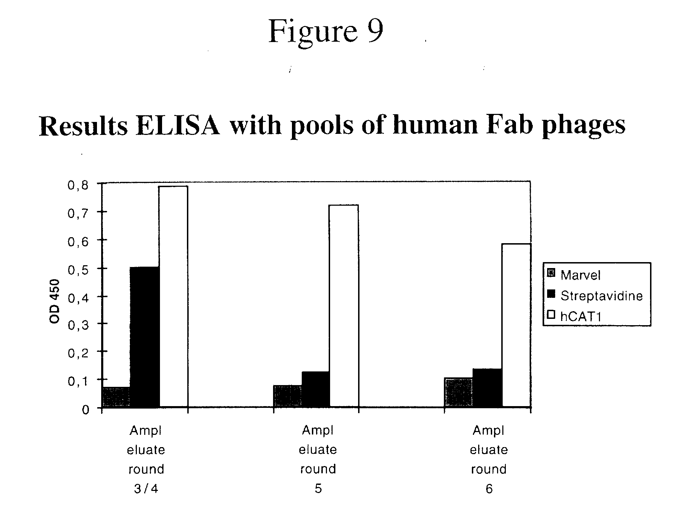

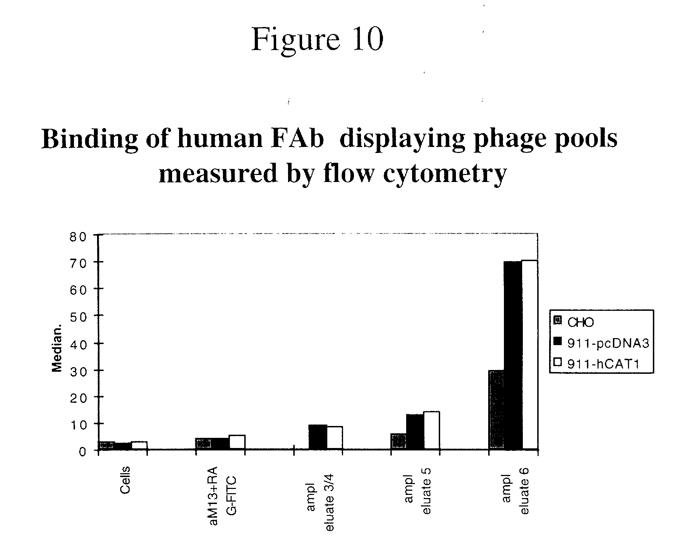

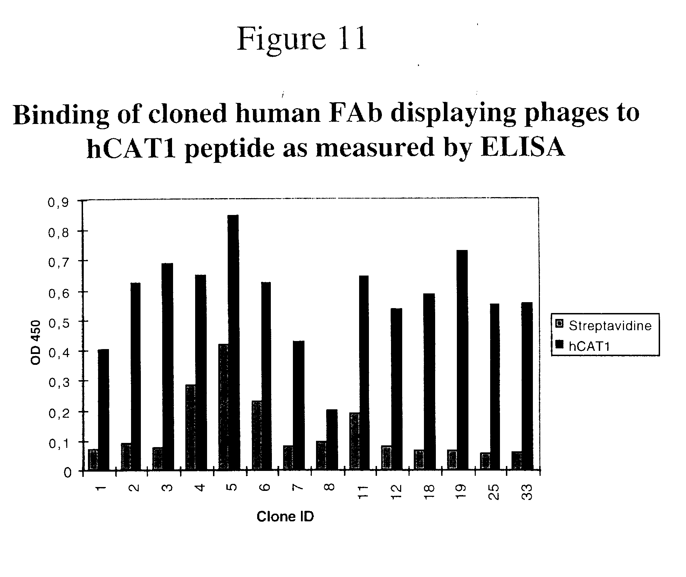

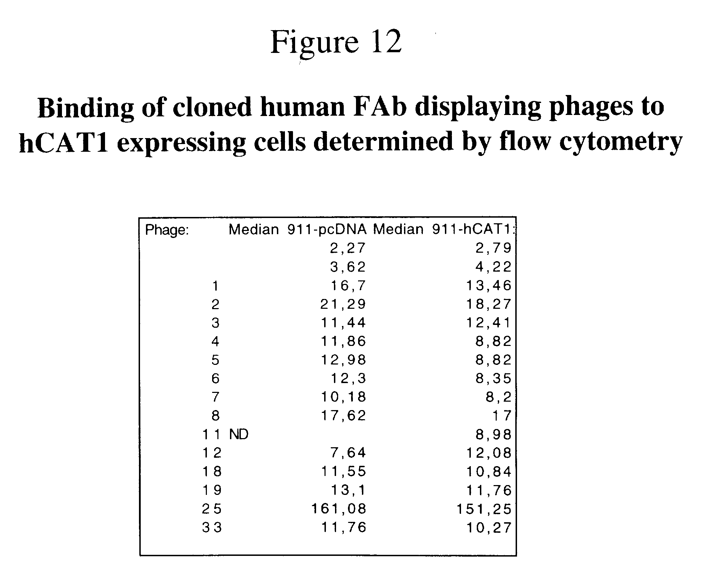

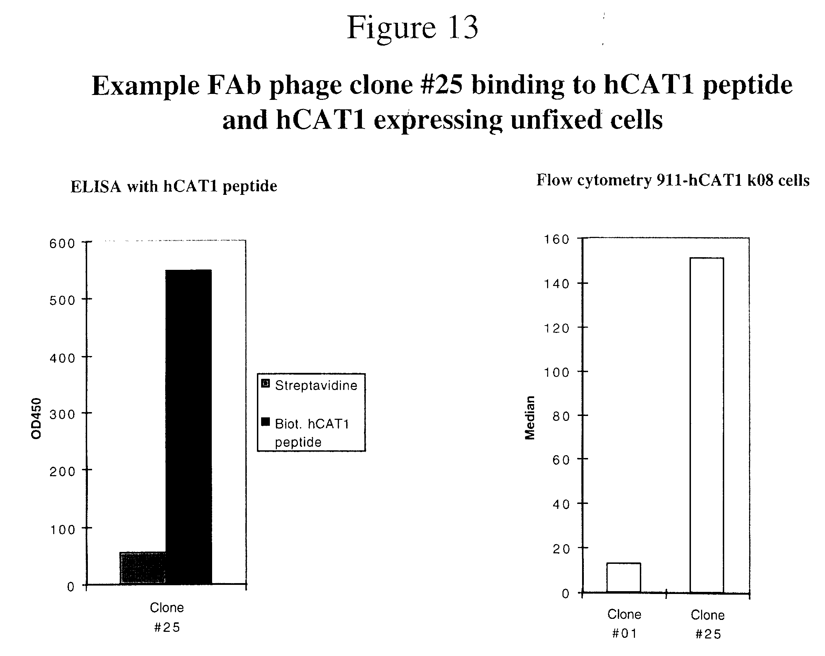

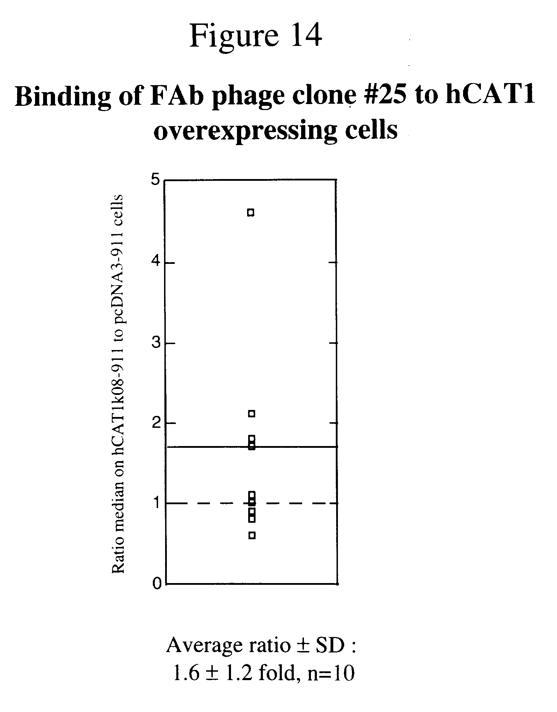

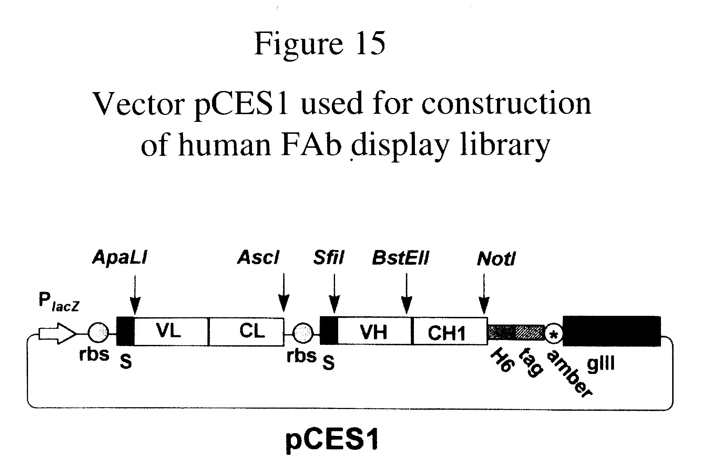

The pools of the last 3 rounds were tested for binding to the biotinylated hCAT1 peptide in a hCAT1 specific ELISA and also for cell binding by flow cytometric analysis (both protocols are described in example 2). After the last round of selection on cells the pool of FAb phages still binds to the biotinylated hCAT1 peptide (figure 9). Flow cytometric analysis showed that this pool also binds to hCAT1 overexpressing cells (figure 10). From this pool 43 clones were analysed by fingerprint analysis and divided into 14 different groups. From each group 1 phage clone was tested for binding to the biotinylated hCAT1 peptide in a hCAT1 specific ELISA and also for cell binding by FACS analysis. Three clones appeared to be streptavidin binders whereas the other 11 clones showed binding to the biotinylated hCAT1 peptide (figure 11). Flow cytometric analysis revealed that only 1 of the 14 clones showed strong binding to hCAT1 overexpressing cells (figure 12). This clone was analyzed in more detail (figures 13,14). Clearly clone #25 binds strongly to the synthetic hCAT1 peptide used and to hCAT1 overexpressing 911 k08 cells. Moreover average fold increased binding of this phage to 911-hCAT1-k08 overexpressing cells over 911-pcDNA3 cells was found to be 1.6 ± 1.2 fold (figure 14). Double strand phagemid DNA was prepared and used to determine the nucleotide and deduced amino acid sequence of the displayed variable heavy and light chains. For a schematic picture of the vector pCES1 in which the library of variable chains was cloned (see figure 15). The hCAT1 binding domains are as expected homologous to human immunoglobulin sequences. The complementarity determining regions (CDRs) are indicated in figure 16.
The sequences of this immunoglobulin can be incorporated in viral or non-viral proteins that mediate binding and entry to cells and thus create gene transfer vehicles that enter cells through hCAT1. The hCAT1 binding human FAbs can also be used to measure expression of hCAT1 on cells that are targets for gene therapy using hCAT1 mediated gene transfer.

### Example 4 Incorporation of hCAT1 binding peptides in ecotropic retroviral envelope

To include hCAT1 binding peptides (see example 2) in the context of an ecotropic murine leukemia viral envelope we used functional display of ecotropic envelope by filamentous phages. We used the construct gpIII/env2 which encodes a fusion protein consisting of a prokaryotic signal peptide and all of the gp70 protein including the variable regions A and B and the polyproline hinge(amino acid residues 34-308) fused to the capsid protein encoded by gene III of m13. Numbering of amino acid sequences was done according to the unprocessed envelope sequence as deposited in the Swiss prot database with accession number P03385 and starting from the viral signal peptide. In table 5 all the oligonucleotides are depicted that are used for insertion of the peptide sequences in retroviral envelope.

Three sites and ways of peptide insertion have been chosen: (1) Insertion at the BstEII site of the ecotropic envelope. (2) Replacement of sequence PFSS (residues 96-99) by each of the 4 the peptides (see table 5). (3) Replacement of sequence LTSLTP (residues 122-127) by each of the 4 peptide sequences peptides (see table 5). The sequences PFSS and LTSLTP are predicted to be on displayed on the outside of the envelope protein as deduced from the structure of crystallised Friend ecotropic envelope (Fass et al., 1997). For the BstEII insertion constructs the two single stranded complementary oligonucleotides were synthesised. At the amino acid sequence level linker amino acids were included at the amino and carboxyterminus of the inserted peptide sequence. These single stranded oligonucleotides were then mixed in equimolar fashion heated to 95 °C and slowly cooled to room temperature to allow hybridisation of the complementary molecules to double stranded DNA. Annealing was followed by BstEII digestion and separation on a 2 % agarose gel run in TAE-buffer. DNA was then excised from the gel and purified using Qiaquick gel extraction kit (Qiagen,Germany). At the same time double stranded phagemid DNA of construct gpIII/env2 was digested and thus linearized with BstEII. Linearized gpIII/env2 DNA was subjected to an incubation with the thermosensitive alkaline phosphatase TSAP (Life technologies), then mixed in various molar ratios with double stranded BstEII digested oligonucleotides encoding any of the 4 hCAT1 binding peptides (see table 5b). Then 1 unit of T4 ligase and T4 ligase buffer supplemented wit 1 mM ATP as added. The ligation mixture was incubated for 1 hour at + 20 °C. The ligation mixtures were then transformed into Max DH5a competent bacteria (Life technologies). Ampicillin resistant colonies were picked and subjected to a PCR with one of the 4 primers in table 5c and primer Ecoenv12 (see table 5). This PCR allows one to determine the nature of the inserted sequence and its orientation. Plasmid DNA of colonies with correct orientation of insert DNA was then isolated using Qiagen columns and sequenced (Baseclear, Leiden) to confirm the complete sequence of the inserts and boundaries plus their orientation.

For the insertion/deletion of hCAT1 binding peptide sequences into gpIII/env2 at the LTSLTP and PFSS positions two fragments were amplified (primary PCR) using Elongase polymerase and the following two pairs of primers: Fragment 1: Ecoenv17 (sense primer, table 5c) plus one of the even numbered oligonucleotides in table 5a. Fragment 2: Ecoenv12 or ecoenv05 (antisense, table 5c) plus an odd numbered primer in table 5a. Fragment 1 harbours at the DNA level the 3' end whereas fragment 2 harbours the peptide insertion at the 5' end. Because both fragments have identical 3' (fragment 1) and 5' ends (fragment 2) they can be used to assemble a full double stranded DNA fragment encompassing the ecotropic envelope sequence between and including part of the ecoenv17 and ecoenv12 oligonucleotide sequences. This is done by first purifying the two fragments from the primary PCR using Qiaquick PCR purification columns to remove all remaining primers followed by doing a PCR using the two overlapping fragments, and primers ecoenv17 and ecoenv12, and all the components necessary for DNA amplification using Elongase. This step results in the assembly of a fragment harbouring the 12 mer hCAT1 binding peptide insertions and result in the deletion of the LTSLTP or PFSS sequence. These fragments are purified and digested with NotI and PinA1 resulting in a DNA fragment of approximately 519 basepairs which was isolated from an agarose gel using Qiagen DNA isolation kit. The 519 basepair fragments were then ligated into a NotI and PinA1 digested gpIII/env2 Surfscript fragment of approximately 4000 basepairs using T4 ligase as described above in example 3. E coli bacteria are then transformed with the ligation mixture, ampicillin colonies picked, plasmid DNA isolated and analyzed for the presence of 519 basepair inserts using Not1 and PinA1 restriction enzymes and DNA agarose gel electrophoresis. Plasmids with appropriate inserts were then further verified by automated DNA sequencing of the inserts (Baseclear, Leiden).

Phages displaying envelope with the various peptide inserts can then be produced and tested for their binding to and entry of hCAT1 expressing cells and compared to phages displaying the gpIII/env2 construct. The hCAT1 binding envelopes can then be used to develop retroviral vectors produced by mammalian cell lines.

### Example 5 Soluble FAb generation and binding to human cells

To prepare soluble FAb fragments of the hCAT1 binding FAb phage clone periplasmic fractions were made from HB2151 bacteria infected with clone # 25 phage. Infected bacteria were grown in LB medium with 2% glucose and 100 mg/ml ampicillin (LBGA) overnight while shaking at 30 °C. The next day the infected cells were diluted 1:100 in 50 ml fresh LBGA and grown at 37 °C until the OD₆₀₀ was 0.8. Bacteria were then pelleted followed by resuspension in 25 ml LB, 100 mg/ml ampicillin and 1 mM IPTG and incubation for 3 hours at 30 °C while shaking vigorously. Then the bacteria were pelleted and resuspended in 1 ml ice-cold PBS followed by a 14-16 hour incubation at 4 °C while rotating. The next day the periplasmic fraction was cleared from bacterial residues by centrifugation: once for 10 minutes at 8000 rpm C (Eppendorf centrifuge #5402) at 4 °C followed by a spin of 10 minutes at 14000 rpm, 4 °C . Then the FAb periplasmic fractions were aliquoted and stored at -20 °C. The presence and expression of FAb fragments was confirmed by doing a dot blot and probing for human kappa light chains with anti-human kappa polyclonal rabbit antibodies (Dako A0191, 1:1000 dilution, 60 minutes) and anti rabbit IgG (H + L) antibodies conjugated with horse radish peroxidase (Biorad, 170-6515, 1:20.000, 60 minutes). Each step was followed by washing 6 times with PBS, 0.05 % Tween 20 (v/v). Final detection of human FAbs was done using ECL staining (Amersham). This revealed the presence of high concentrations of soluble FAb fragment of hCAT1 binding clone # 25. Dilutions of antibodies were made in PBS, 0.5 % BSA (w/v), 0.05 % Tween 20 (v/v).

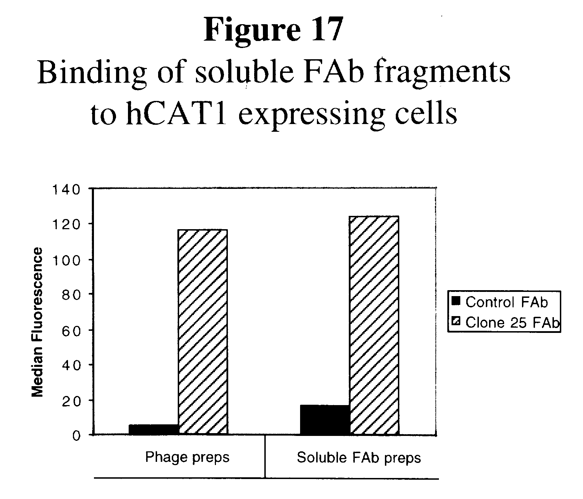

The FAb fractions made as described above were then used to perform flow cytometric analyses in 911-hCAT1-k08 cells expressing hCAT1 and compared to phages displaying clone 25 (see example 3) (figure 17). For this purpose cells were incubated with 100 ml periplasmic fraction of clone 25 or control clone for 1 hour at room temperature followed by washing twice with PBS, 0.1 % BSA and incubation with 500 times diluted anti-human kappa light chain antibodies (see above) for 30 minutes at room temperature. This was followed by washing twice with PBS/0.1 % BSA and a 30 minute room temperature incubation with goat-anti-rabbit immunoglobulin antibodies conjugated with phycoerythrin (diluted in 1:20 in PBS/0.1 % BSA, Sigma P9795) and measurement in a flow cytometer. Detection of phage binding was done as described under example 2.

Clearly FAb preparations of clone 25 bind to hCAT1 expressing cells whereas FAb fragments of an irrelevant CHO cell binding clone did not. The results are very similar to the results observed with phages displaying FAb clone 25 (figure 17). Compared to phages displaying hCAT1 binding FAb 25, FAb fragments of clone 25 facilitate the measurement of hCAT1 in a multiparameter setting such as CD34⁺ or CD34⁺lin⁻ cells.

### Example 6 Generation of adenoviral vectors displaying hCAT1 binding peptide sequences in their fiber sequences

Adenoviral vectors displaying hCAT1 binding sequences such as those described in example 2 and 3 are useful in stem cell gene therapy but also for other applications where a broad tropism is desired such as other gene therapy applications where local administration of the vector is used but where the primary tissue or cells are difficult to transduce with an adenoviral vector. Also these vectors can be used in functional genomics applications where gene or nucleic acid libraries are built in an adenoviral vector system and where transduction of as many cell types as possible is desired. For these purposes we included the peptide sequences describe in examples 2 and 3 in the H-I loop of adenovirus with serotype 5 (Xia et al, Structure, 1994 Dec 15;2(12):1259-70).

### Generation of adenovirus template clones lacking DNA sequences encoding for adenoviral fiber

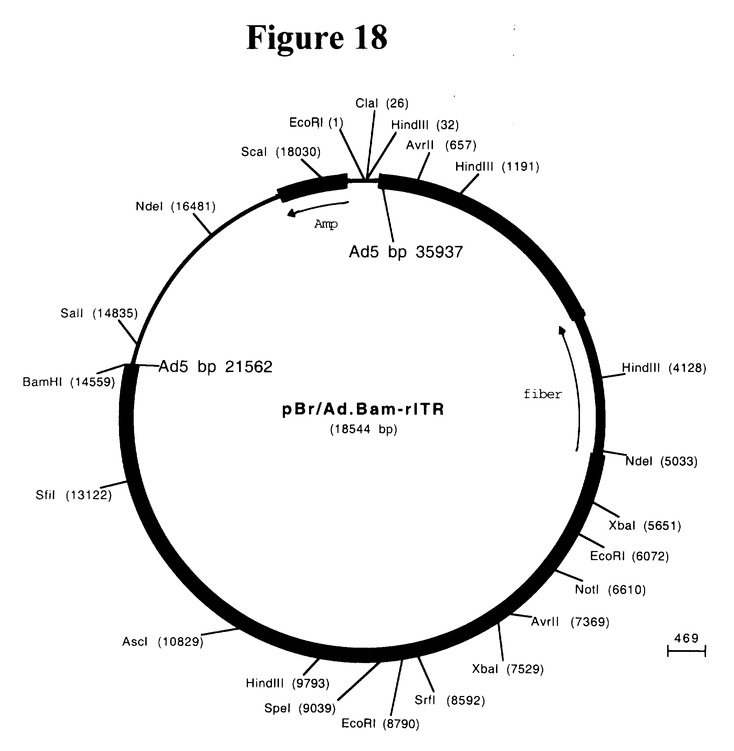

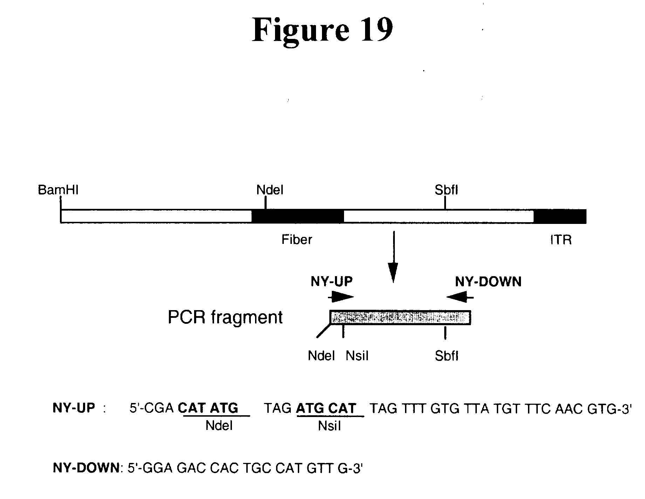

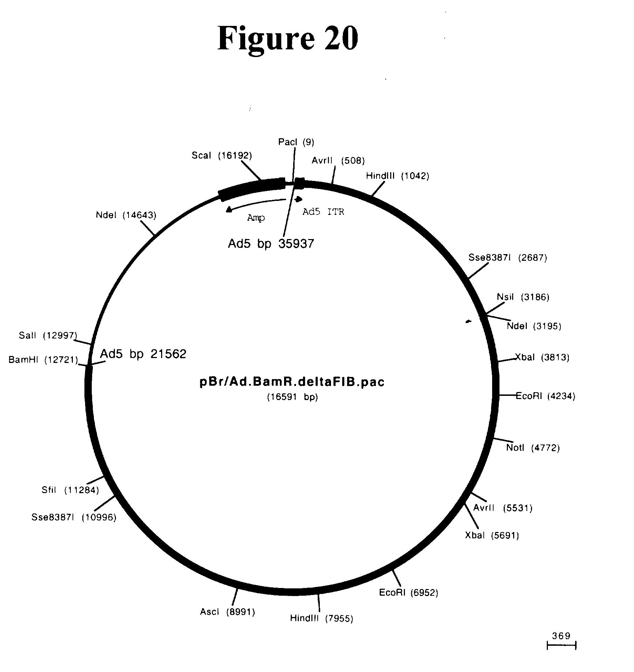

The fiber coding sequence of adenovirus serotype 5 is located between nucleotides 31042 and 32787. To remove the adenovirus serotype 5 DNA encoding fiber we started with construct pBr/Ad.Bam-rITR (Figure 18; ECACC deposit P97082122). From this construct the first step was the removal of a NdeI site. pBr322 plasmid DNA was digested with NdeI after which protruding ends were filled using Klenow enzyme. This pBr322 plasmid was then re-ligated, digested with NdeI and transformed into *E.coli* DH5α. The obtained pBr/ΔNdeI plasmid was digested with ScaI and SalI and the resulting 3198 bp vector fragment was ligated to the 15349 bp ScaI-SalI fragment derived from pBr/Ad.Bam-rITR, resulting in plasmid pBr/Ad.Bam-rITRΔNdeI which hence contained a unique NdeI site. Next a PCR was performed with oligonucleotides "NY-up" and "NY-down" (figure 19). During amplification, both a NdeI and a NsiI restriction site were introduced to facilitate cloning of the amplified fiber DNAs. Amplification consisted of 25 cycles of each 45 sec. at 94 °C, 1 min. at 60 °C, and 45 sec. at 72 °C. The PCR reaction contained 25 pmol of oligonucleotides NY-up and NY-down, 2mM dNTP, PCR buffer with 1.5 mM MgCl₂, and 1 unit of Elongase heat stable polymerase (Life Technologies, Breda, The Netherlands). One-tenth of the PCR product was run on an agarose gel which demonstrated that the expected DNA fragment of about 2200 bp was amplified. This PCR fragment was subsequently purified using the Geneclean kit system for DNA purification (Bio101 Inc.) Then, both the construct pBr/Ad.Bam-rITRΔNdeI as well as the PCR product were digested with restriction enzymes NdeI and SbfI. The PCR fragment was subsequently cloned using T4 ligase enzyme into the NdeI and SbfI sites thus generating pBr/Ad.BamRΔFib. This plasmid allows insertion of any PCR amplified fiber sequence through the unique NdeI and NsiI sites that are inserted in place of the removed fiber sequence. Viruses can be generated by a double homologous recombination in packaging cells described *infra* using an adapter plasmid, construct pWe/Ad.AflII-EcoRI (described below) digested with PacI and EcoRI and a pBr/Ad.BamRAFib construct in which heterologous fiber sequences have been inserted. To increase the efficiency of virus generation, the construct pBr/Ad.BamRΔFib was modified to generate a PacI site flanking the right ITR. Hereto, pBr/Ad.BamRΔFib was digested with AvrII and the 5 kb adenoviral fragment was isolated and introduced into the vector pBr/Ad.Bam-rITR.pac#8 (ECACC deposit P97082121)_replacing the corresponding AvrII fragment. The resulting construct was named (pBr/Ad.BamR.ΔFIB.pac).
Once a heterologous fiber sequence is introduced in pBr/Ad.BamRΔFib.pac, the fiber modified right hand adenovirus clone may be introduced into a large cosmid clone. Such a large cosmid clone allows generation of adenovirus by only one homologous recombination making the process extremely efficient.

### Generation of chimeric adenoviral DNA constructs

For the insertion of hCAT1 binding peptide sequences in the HI loop of Adenovirus serotype 5 two fragments were amplified (primary PCR) using Elongase polymerase and the following two pairs of primers: Fragment 1: NdeIad5-1 (sense primer, table 6 ) plus one of the AS (odd numbered) primers in table 6. Fragment 2: NsiIAd5-1 (antisense primer, table 6) plus one of the sense (even numbered) primers in table 6.
Amplification for all PCRs consisted of a single hot start of 4 min at 94 °C followed by 30 cycles of each 30 sec. at 94 °C, 30 sec. at 49 °C, and 2 min. at 68 °C ending with a 7 minute period at 68 °C. The PCR reaction contained 25 pmol of oligonucleotides NY-up and NY-down, 2mM dNTP, PCR buffer with 1.5 mM MgCl₂, and 1 unit of Elongase heat stable polymerase (Life Technologies, Breda, The Netherlands).
Fragment 1 harbours at the DNA level the 3' end the hCAT1 binding sequences whereas fragment 2 harbours the peptide insertions at the 5' end. Since both fragments have now overlapping 3' (fragment 1) and 5' ends (fragment 2) they can be used to assemble a full double stranded DNA fragment encompassing one of the insertions (designated in hCAD1, hCAD2, hCAD3 or hCAD4 for hCAT1 binding and Adenovirus).

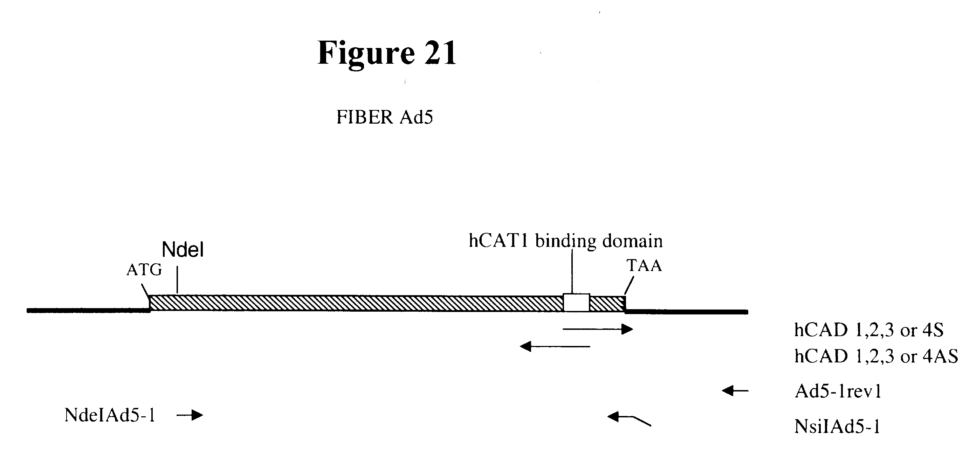

Figure 21 schematically depicts the method and primers used to generate the primary PCR products and assembly PCR. This is done by first running the crude PCR products on a 1 % agarose gel (TAE) isolating the two fragments from the primary PCR out of a gel-slice using a Qiagen DNA isolation kit to remove all remaining primers and original template. This procedure was followed by a second "assembly" PCR using the two overlapping fragments, and two outer primers NdeIad5-1 (5') and NsiIAd5-1 (3'), and all the components necessary for DNA amplification using Elongase. This step results in the assembly of a fragment harbouring the 12 mer hCAT1 binding peptide insertions and linker.
Oligonucleotide Ad5-1rev1 was designed for an alternative assembly PCR strategy. The primary PCR of fragment 2 results in a PCR product of 633 bp harbouring the 12 mer hCAT1 binding peptide insertions. Assembly PCR using this fragment and the 5' fragments generated as described above ("fragment 1") results in an assembled fragment of 2271 bp which is easier to distinguish from the 5' primary fragment 1. The secondary assembly fragments were purified and digested with NdeI and NsiI resulting in a DNA fragment of 1736 basepairs. This fragment was isolated from a 1 % agarose gel (TAE) using a Qiagen DNA isolation kit. The 1736 basepair fragments were then ligated into a NdeI and NsiI digested fragment of approximately 16853 basepairs derived from pBr/Ad.BamRΔ Fib.pac using T4 ligase. Electrocompetent E. coli bacteria (XL1 blue, Stratagene, >10¹⁰/microgram plasmid DNA) were then electroporated with the ligation mixture, ampicillin resistant colonies picked, plasmid DNA isolated and analyzed for the presence of 1736 basepair inserts using NdeI and NsiI restriction enzymes and DNA agarose gel electrophoresis. Plasmids with appropriate inserts were then further verified by automated DNA sequencing of the inserts.

### Generation of recombinant adenovirus chimeric for fiber protein

To enable efficient generation of chimeric viruses, transfection using pClipsal/lacZ, pWE/Ad.AflII-Eco and pBr/Ad.Bam-rITR.PacI-hCAD1,2,3 or 4 fib5. pBr/Ad.BamrITR.PacI-hCAD1,2,3 or 4 fib5 all have a PacI site near the right ITR that enables the ITR to be separated from the vector sequences and allows efficient initiation of adenoviral replication after the complete vector molecule has been generated after double homologous recombination. The plasmid pClipsal/lacZ was generated as follows

### Generation of pAd5/ClipsalLacZ

First, a PCR fragment was generated from pZipΔmo+PyF101(N⁻) template DNA (described in PCT/NL96/00195) with the following primers: LTR-1: 5'-CTG TAC GTA CCA GTG CAC TGG CCT AGG CAT GGA AAA ATA CAT AAC TG-3' and LTR-2: 5'-GCG GAT CCT TCG AAC CAT GGT AAG CTT GGT ACC GCT AGC GTT AAC CGG GCG ACT CAG TCA ATC G-3'. Pwo DNA polymerase (Boehringer Mannheim) was used according to manufacturers protocol with the following temperature cycles: once 5' at 95°C; 3' at 55°C; and 1' at 72°C, and 30 cycles of 1' at 95°C, 1' at 60°C, 1' at 72°C, followed by once 10' at 72°C. The PCR product was then digested with BamHI and ligated into pMLP10 (Levrero et al., 1991) vector digested with PvuII and BamHI, thereby generating vector pLTR10. This vector contains adenoviral sequences from bp 1 up to bp 454 followed by a promoter consisting of a part of the Mo-MuLV LTR having its wild-type enhancer sequences replaced by the enhancer from a mutant polyoma virus (PyF101). The promoter fragment was designated L420. Next, the coding region of the murine HSA gene was inserted. pLTR10 was digested with BstBI followed by Klenow treatment and digestion with NcoI. The HSA gene was obtained by PCR amplification on pUC18-HSA (Kay et al., 1990) using the following primers: HSA1, 5'-GCG CCA CCA TGG GCA GAG CGA TGG TGG C-3' and HSA2, 5'-GTT AGA TCT AAG CTT GTC GAC ATC GAT CTA CTA ACA GTA GAG ATG TAG AA-3'. The 269 bp amplified fragment was subcloned in a shuttle vector using the NcoI and BglII sites. Sequencing confirmed incorporation of the correct coding sequence of the HSA gene, but with an extra TAG insertion directly following the TAG stop codon.The coding region of the HSA gene, including the TAG duplication was then excised as a NcoI(sticky)-SalI(blunt) fragment and cloned into the 3.5 kb NcoI(sticky)/BstBI(blunt) fragment from pLTR10, resulting in pLTR-HSA10.
Finally, pLTR-HSA10 was digested with EcoRI and BamHI after which the fragment containing the left ITR, packaging signal, L420 promoter and HSA gene was inserted into vector pMLPI.TK (described in WO 97/00326) digested with the same enzymes and thereby replacing the promoter and gene sequences. This resulted in the new adapter plasmid pAd/L420-HSA that contains convenient recognition sites for various restriction enzymes around the promoter and gene sequences. SnaBI and AvrII can be combined with HpaI, NheI, KpnI, HindIII to exchange promoter sequences, while the latter sites can be combined with the ClaI or BamHI sites 3' from HSA coding region to replace genes in this construct.

Another adapter plasmid that was designed to allow easy exchange of nucleic acid molecules was made by replacing the promoter, gene and poly A sequences in pAd/L420-HSA with the CMV promoter, a multiple cloning site, an intron and a poly-A signal. For this purpose, pAd/L420-HSA was digested with AvrII and BglII followed by treatment with Klenow to obtain blunt ends. The 5.1 kb fragment with pBr322 vector and adenoviral sequences was isolated and ligated to a blunt 1570 bp fragment from pcDNA1/amp (Invitrogen) obtained by digestion with HhaI and AvrII followed by treatment with T4 DNA polymerase. This adapter plasmid was named pCLIP.

To enable removal of vector sequences from the left ITR in pAd5/Clip (described in example 2B), this plasmid was partially digested with EcoRI and the linear fragment was isolated. An oligo of the sequence 5' TTAAGTCGAC-3' was annealed to itself resulting in a linker with a SalI site and EcoRI overhang. The linker was ligated to the partially digested pAd5/Clip vector and clones were selected that had the linker inserted in the EcoRI site 23 bp upstream of the left adenovirus ITR in pAd5/Clip resulting in pAd5/Clipsal.

The adapter plasmid pAd5/Clipsal.LacZ was generated as follows: The E.coli LacZ gene was amplified from the plasmid pMLP.nlsLacZ (EP 95-202 213) by PCR with the primers 5 'GGGGTGGCCAGGGTACCTCTAGGCTTTTGCAA and S'GGGGGGATCCATAAACAAGTTCAGAATCC. The PCR reaction was performed Ex Taq (Takara) according to the suppliers protocol at the following amplification program: 5 minutes 94°C, 1 cycle; 45 seconds 94°C and 30 seconds 60°C and 2 minutes 72°C, 5 cycles; 45 seconds 94°C and 30 seconds 65°C and 2 minutes 72°C, 25 cycles; 10 minutes 72; 45 seconds 94°C and 30 seconds 60°C and 2 minutes 72°C, 5 cycles, I cycle. The PCR product was subsequently digested with KpnI and BamHI and the digested DNA fragment was ligated into KpnI/BamHI digested pcDNA3 (Invitrogen), giving rise to pcDNA3.nlsLacZ. Next, the plasmid pAd5/Clipsal was digested with SpeI. The large fragment containing part of the 5' part CMV promoter and the adenoviral sequences was isolated. The plasmid pcDNA3.nlsLacZ was digested with SpeI and the fragment containing the 3'part of the CMV promoter and the lacZ gene was isolated. Subsequently, the fragments were ligated, giving rise to pAd/Clipsal.LacZ.

pWE/Ad.AflII-EcoRI was generated as follows. Cosmid vector pWE15 (Clontech) was used to clone larger Ad5 inserts. First, a linker containing a unique PacI site was inserted in the EcoRI sites of pWE15 creating pWE.pac. pWE.pac was digested with ClaI and 5' protruding ends were filled using Klenow enzyme. The DNA was then digested with PacI and isolated from agarose gel. pWE/Ad.AflII-rITR (ECACC deposit P97082116) was digested with EcoRI and after treatment with Klenow enzyme digested with PacI. The large 24 kb fragment containing the adenoviral sequences was isolated from agarose gel and ligated to the ClaI-digested and blunted pWE.pac vector using the Ligation Express^{tm} kit from Clontech. After transformation of Ultracompetent XL10-Gold cells from Stratagene, clones were identified that contained the expected insert. pWE/AflII-EcoRI contains Ad5 sequences from bp 3534-27336.

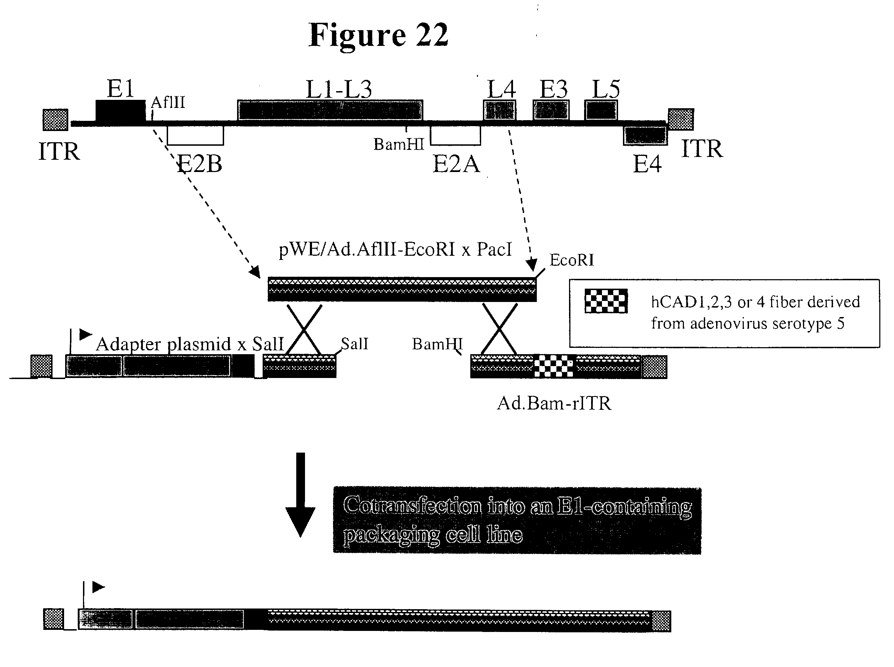

Before transfection the three necessary DNA constructs were treated as follows: pClipsal/lacZ was linearized by digestion with SalI; pWE/Ad.AflII-Eco was digested with PacI and EcoRI; The four different pBr/Ad.Bam-rITR.PacI-hCAD1,2,3 or 4 fib5 constructs were digested with BamHI and PacI. These three digested DNA preparations were transfected into PER.C6 to generate recombinant adenovirus. Figure **22** schematically depicts the method and fragments used to generate the chimeric viruses. Alternatively other adapter fragments containing a different marker gene or stronger promoter could be used.
For transfection, 2µg of pCLIPsal/lacZ and 4 µg total of pWE/Ad.AflII-Eco and 2 µg of one of the four different pBr/Ad.Bam-rITR.PacI-hCAD1,2,3 or 4 fib5 constructs were diluted in serum free DMEM to 100 µl total volume. To this DNA suspension 100 µl 2.5 times diluted lipofectamine (Life Technologies) in serum-free DMEM medium was added. After 30 minutes at room temperature the DNA-lipofectamine complex solution was added to 2.3 ml of serum-free DMEM. This mixture was then added to a culture flask with a surface area of 25 cm² (T25). This T25 flask was seeded with PER.C6 cells 24-hours prior to transfection at a density of 3.5 x 10⁶ cells/flask. Two hours later, the DNA-lipofectamine complex containing medium was diluted by the addition of 2.5 ml DMEM supplemented with 10 % foetal bovine serum. Again 24 hours later the medium was replaced by fresh DMEM supplemented with 10 % foetal bovine serum. On day 2 after transfection the cells were passed to a tissue culture flask (T80) with a surface area of 80 cm². Cells were subsequently cultured for 4-14 days. During this period the medium was replaced with fresh medium upon medium depletion and or reaching confluency. At full CPE the virus was harvested by one freeze/thaw cycle. Part of the supernatant was used to reinfect a T80 flask with PER.C6 cells. This propagation resulted in viruses displaying hCAT1 binding sequences carrying a lacZ as a marker gene These virus-preparations were then used to transduce hCAT1 expressing cells including human and non-human primate hemopoietic stem cells, such as human smooth muscle cells, human chorion villi cells, human primary tumour cells, human and mouse fibroblasts, human synoviocytes and compared to the transduction efficiency of adenovirus 5 vector without the hCAT1 binding peptide insertions but with the introduced NdeI and NsiI sites.

For those skilled in the art modifications and variations of the methods and materials described herein will be obvious. As an example but by no means intended as a limitation other desired therapeutic transgenes can be incorporated in adenoviral vectors displaying the hCAT1 binding peptide sequences described herein such as the cDNA for endothelial nitric oxide synthase or libraries of nucleic sequences either as pools or as collections of individual clones made either manually or using automated procedures.

### REFERENCES

Albritton, L.M., Kim, J.W., Tseng, L. and Cunningham, J.M. (1993) Envelope-binding domain in the cationic amino acid transporter determines the host range of ecotropic murine retroviruses. J Virol 67(4), 2091-2096.

Albritton, L.M., Tseng, L., Scadden, D. and Cunningham, J.M. (1989) A putative murine ecotropic retrovirus receptor gene encodes a multiple membrane-spanning protein and confers susceptibility to virus infection. Cell 57(4), 659-666. Battini, J.L., Heard, J.M. and Danos, O. (1992) Receptor choice determinants in the envelope glycoproteins of amphotropic, xenotropic, and polytropic murine leukemia viruses. J-Virol 66(3), 1468-75.

Chomczynski, P. and Sacchi, N. (1987) Single-step method of RNA isolation by acid guanidinium thiocyanate- phenolchloroform extraction. Anal Biochem 162(1), 156-9.

Closs, E.I., Rinkes, I.H., Bader, A., Yarmush, M.L. and Cunningham, J.M. (1993) Retroviral infection and expression of cationic amino acid transporters in rodent hepatocytes. J Virol 67(4), 2097-102.

Demarquoy, J. (1993) Retroviral-mediated gene therapy for the treatment of citrullinemia. Transfer and expression of argininosuccinate synthetase in human hematopoietic cells. Experientia 49(4), 345-8.

Fass, D., Davey, R.A., Hamson, C.A., Kim, P.S., Cunningham, J.M. and Berger, J.M. (1997) Structure of a murine leukemia virus receptor-binding glycoprotein at 2.0 angstrom resolution. Science 277(5332), 1662-6.

Gordon, E.M. and Anderson, W.F. (1994) Gene therapy using retroviral vectors. Curr Opin Biotechnol 5(6), 611-6. Gunzburg, W.H. and Salmons, B. (1996) Development of retroviral vectors as safe, targeted gene delivery systems. J Mol Med 74(4), 171-82.

Havenga, M., Hoogerbrugge, P., Valerio, D. and van Es, H.H.G. (1997) Retroviral stem cell gene therapy. Stem cells 15(3), 162-179.

Hoogerbrugge, P.M., van Beusechem, V.W., Fischer, A., Debree, M., Le Deist, F., Perignon, J.L., Morgan, G., Gaspar, B., Fairbanks, L.D., Skeoch, C.H., Mosely, A., Harvey, M., Levinskey, R.J. and Valerio, D. (1996) Bone marrow gene transfer in three patients with adenosine deaminase deficiency. Gene Therapy 3, 179-183.

Januszeski, M.M., Cannon, P.M., Chen, D., Rozenberg, Y. and Anderson, W.F. (1997) Functional analysis of the cytoplasmic tail of Moloney murine leukemia virus envelope protein. J Virol 71(5), 3613-9.

Kafri, T., Blomer, U., Peterson, D.A., Gage, F.H. and Verma, I.M. (1997) Sustained expression of genes delivered directly into liver and muscle by lentiviral vectors. Nat Genet 17(3), 314-7.

Kavanaugh, M.P., Miller, D.G., Zhang, W., Law, W., Kozak, S.L., Kabat, D. and Miller, A.D. (1994) Cell-surface receptors for gibbon ape leukemia virus and amphotropic murine retrovirus are inducible sodium-dependent phosphate symporters. Proc-Natl-Acad-Sci-U-S-A 91(15), 7071-5 issn: 0027-8424.

Kim, J.W., Closs, E.I., Albritton, L.M. and Cunningham, J.M. (1991) Transport of cationic amino acids by the mouse ecotropic retrovirus receptor. Nature 352(6337), 725-728.

Kim, V.N., Mitrophanous, K., Kingsman, S.M. and Kingsman, A.J. (1998) Minimal requirement for a lentivirus vector based on human immunodeficiency virus type 1. J Virol 72(1), 811-6.

Knaan-Shanzer, S., Valerio, D. and van Beusechem, V.W. (1996) Cell cycle state, response to hemopoietic growth factors and retroviral vector-mediated transduction of human hemopoietic stem cells. Gene Ther 3(4), 323-333.

Krasnykh, V., Dmitriev, I., Mikheeva, G., Miller, C.R., Belousova, N. and Curiel, D.T. (1998) Characterization of an adenovirus vector containing a heterologous peptide epitope in the HI loop of the fiber knob. J Virol 72(3), 1844-52.

MacKrell, A.J., Soong, N.W., Curtis, C.M. and Anderson, W.F. (1996) Identification of a subdomain in the Moloney murine leukemia virus envelope protein involved in receptor binding. J-Virol 70(3), 1768-74.

Malhotra, S., Scott, A.G., Zavorotinskaya, T. and Albritton, L.M. (1996) Analysis of the murine ecotropic leukemia virus receptor reveals a common biochemical determinant on diverse cell surface receptors that is essential to retrovirus entry. J Virol 70(1), 321-326.

Masuda, M., Hanson, C.A., Alvord, W.G., Hoffman, P.M., Ruscetti, S.K. and Masuda, M. (1996a) Effects of subtle changes in the SU protein of ecotropic murine leukemia virus on its brain capillary endothelial cell tropism and interference properties. Virology 215(2), 142-51 Issn: 0042-6822.

Masuda, M., Masuda, M., Hanson, C.A., Hoffman, P.M. and Ruscetti, S.K. (1996b) Analysis of the unique hamster cell tropism of ecotropic murine leukemia virus PVC-211. J Virol 70(12), 8534-8539.

McClure, M.O., Sommerfelt, M.A., Marsh, M. and Weiss, R.A. (1990) The pH independence of mammalian retrovirus infection. J Gen Virol 71(Pt 4), 767-773.

McGuiness, B.T., Walter, G., FitzGerald, K., Schuler, P., Mahoney, W., Duncan, A.R. and Hoogenboom, H.R. (1996) Phage diabody repertoires for selection of large numbers of bispecific antibody fragments. Nat Biotechnol 14, 1149-1154.

Miller, D.G. and Miller, A.D. (1994) A family of retroviruses that utilize related phosphate transporters for cell entry. J-Virol 68(12), 8270-6 issn: 0022-538x.

Miyoshi, H., Takahashi, M., Gage, F.H. and Verma, I.M. (1997) Stable and efficient gene transfer into the retina using an HIV-based lentiviral vector. Proc Natl Acad Sci U S A 94(19), 10319-23.

Naldini, L., Blomer, U., Gage, F.H., Trono, D. and Verma, I.M. (1996a) Efficient transfer, integration, and sustained long-term expression of the transgene in adult rat brains injected with a lentiviral vector. Proc Natl Acad Sci U S A 93(21), 11382-8.

Naldini, L., Blomer, U., Gallay, P., Ory, D., Mulligan, R., Gage, F.H., Verma, I.M. and Trono, D. (1996b) In vivo gene delivery and stable transduction of nondividing cells by a lentiviral vector [see comments]. Science 272(5259), 263-7.

Orlic, D., Girard, L.J., Jordan, C.T., Anderson, S.M., Cline, A.P. and Bodine, D.M. (1996) The level of mRNA encoding the amphotropic retrovirus receptor in mouse and human hematopoietic stem cells is low and correlates with the efficiency of retrovirus transduction. Proc Natl Acad Sci U S A 93(20), 11097-11102.

Poeschla, E., Corbeau, P. and Wong-Staal, F. (1996) Development of HIV vectors for anti-HIV gene therapy. Proc Natl Acad Sci U S A 93(21), 11395-9.

Rizvi, T.A. and Panganiban, A.T. (1992) Simian immunodeficiency virus vectors: replication and pseudotyping. J Med Primatol 21(2-3), 69-73.

Skov, H. and Andersen, K.B. (1993) Mutational analysis of Moloney murine leukaemia virus surface protein gp70. J-Gen-Virol 74 (Pt 4), 707-14.

Sullivan, D.E., Dash, S., Du, H., Hiramatsu, N., Aydin, F., Kolls, J., Blanchard, J., Baskin, G. and Gerber, M.A. (1997) Liver-directed gene transfer in non-human primates. Hum Gene Ther 8(10), 1195-206.

Thomas, A., Gray, K.D. and Roth, M.J. (1997) Analysis of mutations within the cytoplasmic domain of the Moloney murine leukemia virus transmembrane protein. Virology 227(2), 305-13.

Van Beusechem, V.W., Bakx, T.A., Kaptein, L.C., Bart-Baumeister, J.A., Kukler, A., Braakman, E. and Valerio, D. (1993) Retrovirus-mediated gene transfer into rhesus monkey hematopoietic stem cells: the effect of viral titers on transduction efficiency. Hum Gene Ther 4(3), 239-47.

van Beusechem, V.W., Kukler, A., Heidt, P.J. and Valerio, D. (1992) Long-term expression of human adenosine deaminase in rhesus monkeys transplanted with retrovirus-infected bone-marrow cells. Proc Natl Acad Sci U S A 89(16), 7640-4.

van Es, H.H.G., Knaan, S., Camphorst, S., Verlinden, S. and Valerio, D. (1996) Expression studies of the amphotropic receptor GLVR2 in mammalian cells and tissues including human CD34+ cells. Cold Spring Harbor Gene therapy meeting, Abstract 309.

van Zeijl, M., Johann, S.V., Closs, E., Cunningham, J., Eddy, R., Shows, T.B. and O'Hara, B. (1994) A human amphotropic retrovirus receptor is a second member of the gibbon ape leukemia virus receptor family. Proc-Natl-Acad-Sci-U-S-A 91(3), 1168-72 issn: 0027-8424.

Vile, R.G., Tuszynski, A. and Castleden, S. (1996) Retroviral vectors. From laboratory tools to molecular medicine. Mol Biotechnol 5(2), 139-58.

von Kalle, C., Kiem, H.P., Goehle, S., Darovsky, B., Heimfeld, S., Torok Storb, B., Storb, R. and Schuening, F.G. (1994) Increased gene transfer into human hematopoietic progenitor cells by extended in vitro exposure to a pseudotyped retroviral vector. Blood 84(9), 2890-7 issn: 0006-4971.

Weiss, R.A. (1996) Retrovirus classification and cell interactions. J Antimicrob Chemother 37 Suppl B, 1-11.

Wilson, C.A., Farrell, K.B. and Eiden, M.V. (1994) Properties of a unique form of the murine amphotropic leukemia virus receptor expressed on hamster cells. J-Virol 68(12), 7697-703 issn: 0022-538x.

Yoshimoto, T., Yoshimoto, E. and Meruelo, D. (1991) Molecular cloning and characterization of a novel human gene homologous to the murine ecotropic retroviral receptor. Virology 185(1), 10-17.

Yoshimoto, T., Yoshimoto, E. and Meruelo, D. (1993) Identification of amino acid residues critical for infection with ecotropic murine leukemia retrovirus. J Virol 67(3), 1310-4 Issn: 0022-538x.

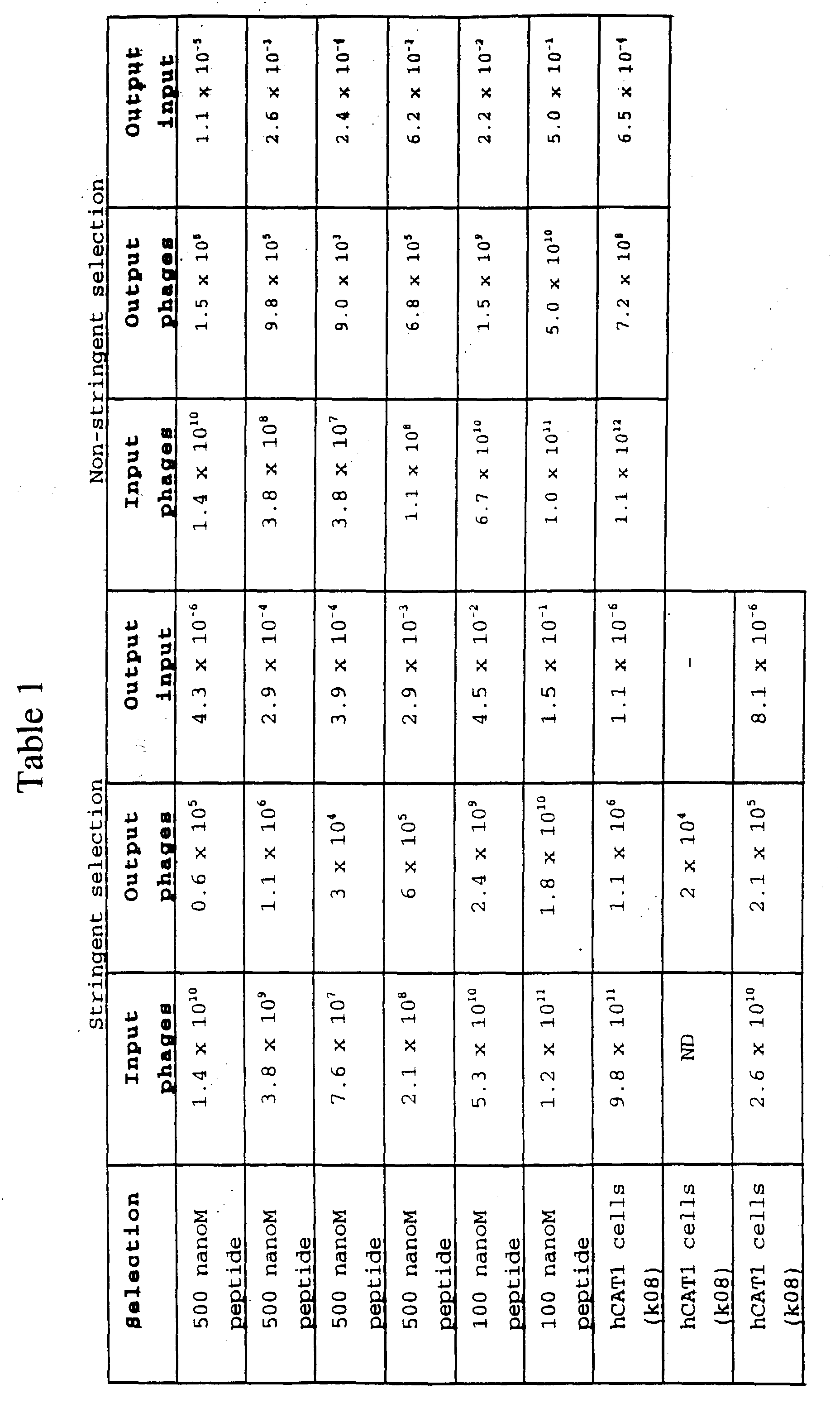

**Table 2**

| **Round** | **Target** | **Insert sequence** | **No of identical clones** |
|---|---|---|---|
| Amplified | None | EQSRPSWQLTPT | 1 |
| library | | QTHQLLRXPPSF | 1 |
| | | YMHEPITPNPVT | 1 |
| | | WHHIPNSAKISL | 1 |
| | | SENLTLMTVLQM | 1 |
| | | NLMPPPVPRLPL | 1 |
| | | TPQGVHYHPNMR | 1 |
| 1 | hCAT1 peptide | ND | |
| 2 | hCAT1 peptide | ND | |
| 3 | hCAT1 peptide | TLNNHTTPPAWN QVVHSPFPTSRP | 1 1 |
| 4 | hCAT1 peptide | ND | |
| 5 | hCAT1 peptide | FEQHNWWDSHPQ NTFDLWLQSVPQ | 1 7 |
| 6 | hCAT1 peptide | FEGCHPQSGLSC | 1 |
| | | FEQHNWWDSHPQ | 1 |
| | | NTFDLWLQSVPQ | 5 |
| | | SVSVGMKPSPRP | 4 |
| 1 | hCAT1 cells | SVSVGMKPSPRP | 4 |
| 2 | hCAT1 cells | SVSVGMKPSPRP | 4 |
| 3 | hCAT1 cells | SVSVGMKPSPRP | 23 |

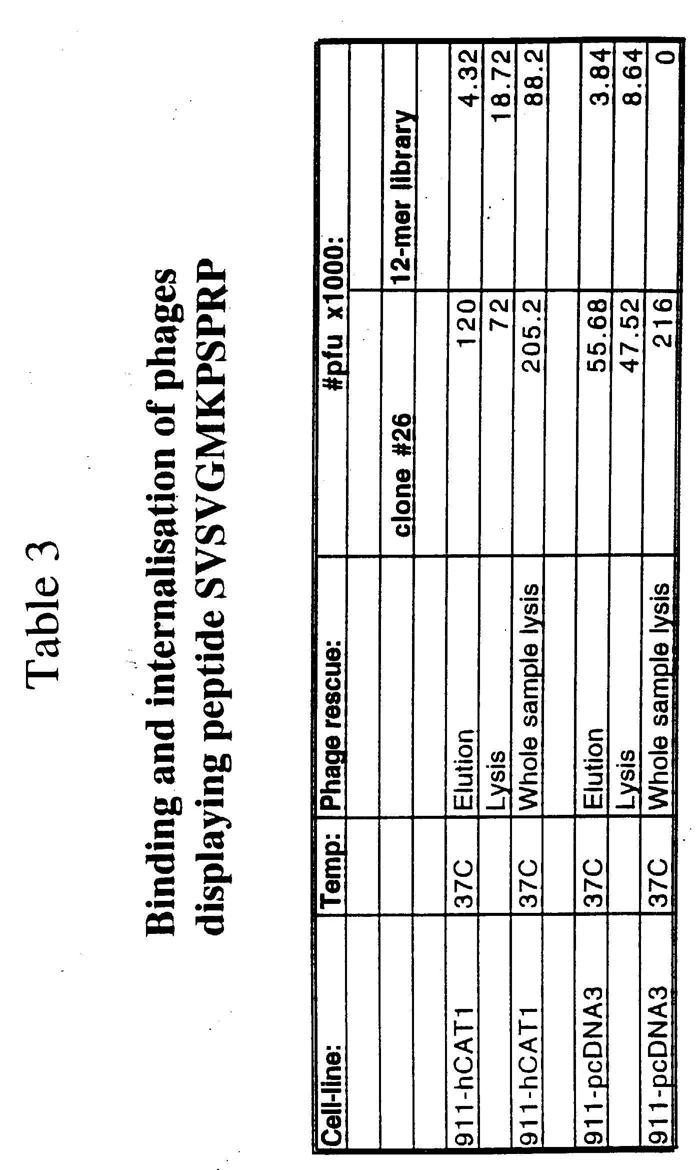

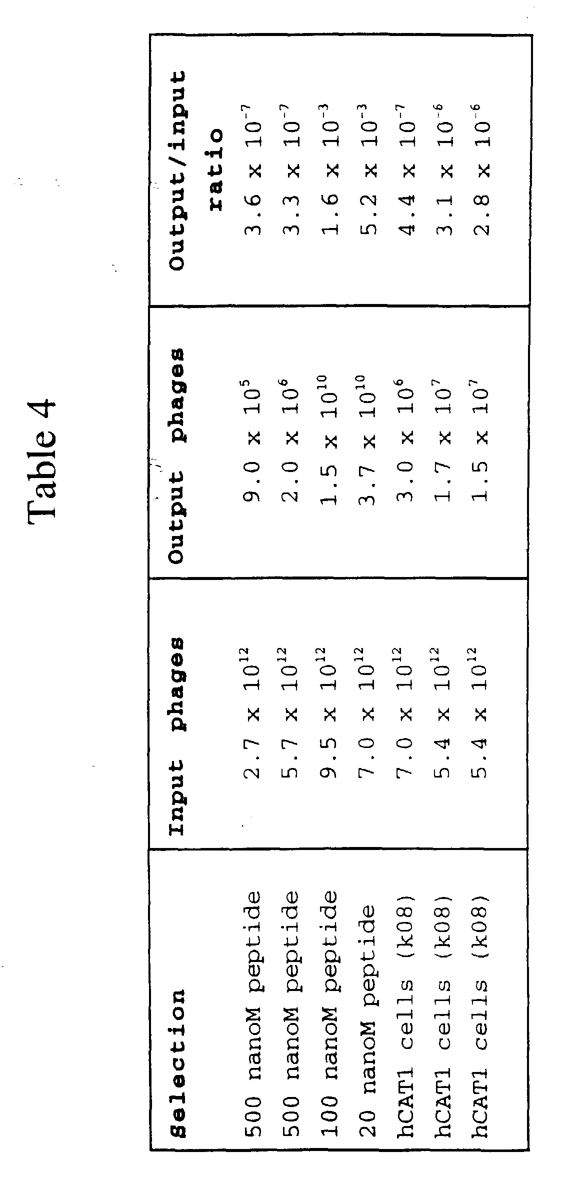

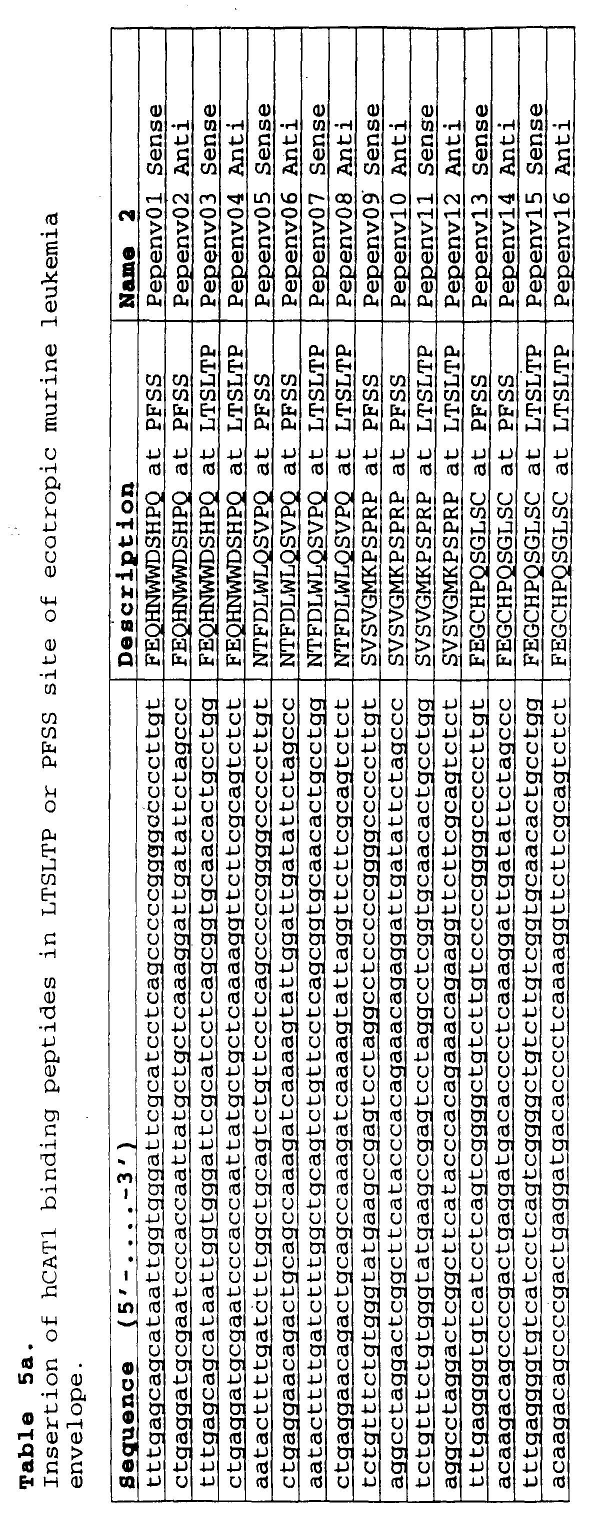

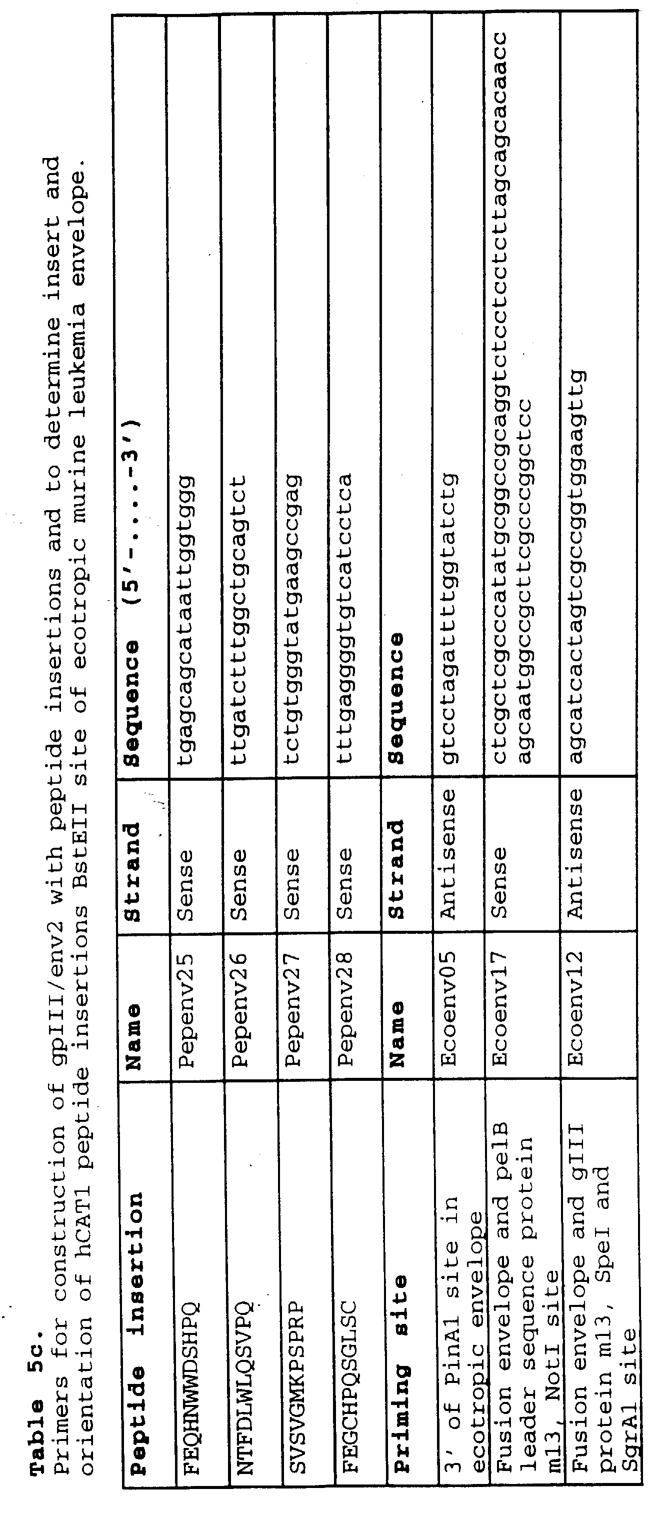

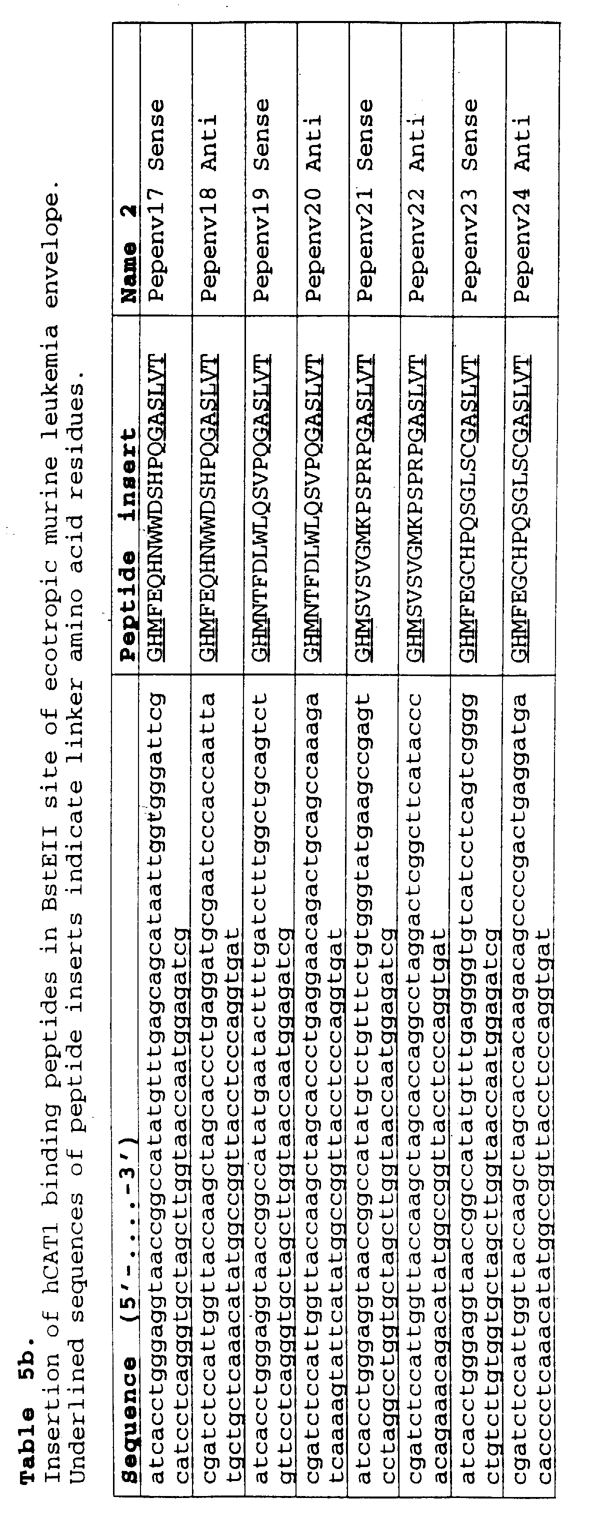

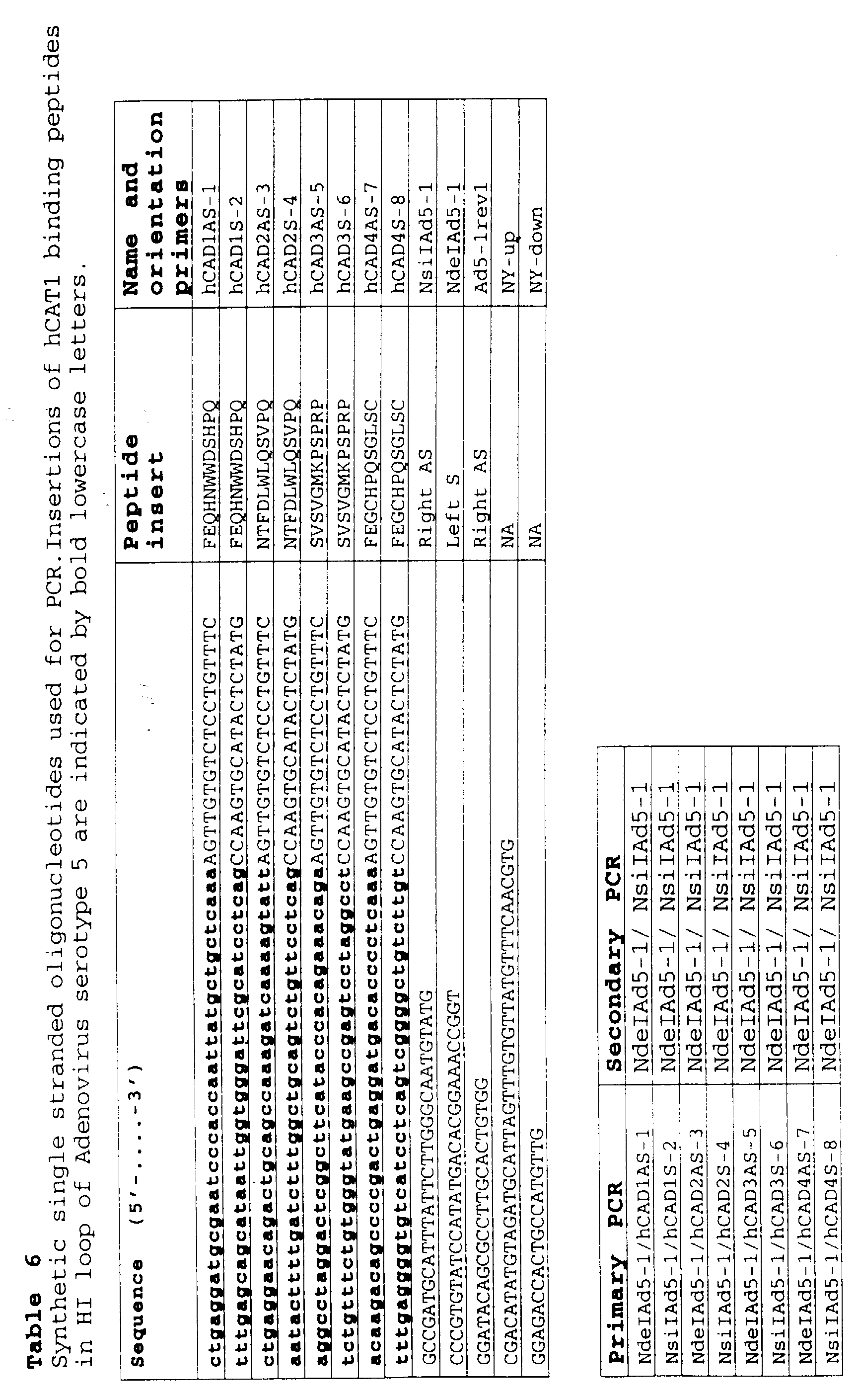

## Claims

1. A virus-like particle or gene delivery vehicle provided with a ligand capable of binding to a human amino acid transporter.

2. A virus-like particle or gene delivery vehicle according to claim 1 for delivery of genes to human cells.

3. A virus-like particle or gene delivery vehicle according to claim 1 or 2 comprising at least one viral protein provided with said ligand.

4. A virus-like particle or gene delivery vehicle according to claim 3 wherein said viral protein comprises an envelope protein.

5. A virus-like particle or gene delivery vehicle according to claim 4 derived from a retrovirus.

6. A virus-like particle or gene delivery vehicle according to claim 3 wherein said viral protein comprises a capsid protein.

7. A virus-like particle or gene delivery vehicle according to claim 6 derived from an adeno virus.

8. A virus-like particle or gene delivery vehicle according to any one of claims 1 to 7 wherein said amino acid transporter is a cationic amino acid transporter.

9. A virus-like particle or gene delivery vehicle according to claim 8 wherein said transporter is a human cationic amino acid transporter-1 (hCAT1).

10. A virus-like particle or gene delivery vehicle according to any one of claims 1 to 9 wherein said ligand comprises an amino acid sequence selected from Table 2, preferably from the last four different sequences of Table 2 or a sequence functionally related thereto.

11. A virus-like particle or gene delivery vehicle according to claim 10 wherein said ligand comprises at least a part of the amino acid sequence SVSVGMKPSPRP.

12. A virus-like particle or gene delivery vehicle according to any one of claims 1 to 9 wherein said ligand comprises a fragment derived from a phage displaying at least one antibody fragment selected for its capacity to bind with said amino acid transporter.

13. A virus-like particle or gene delivery vehicle according to claim 12 wherein said antibody fragment comprises an amino acid sequence as shown in Figure 16 or an amino acid sequence functionally equivalent thereto.
